Europäisches Patentamt

European Patent Office

Office européen des brevets

(19)

(11) Publication number: **0 456 507 A2**

(12) # EUROPEAN PATENT APPLICATION

(21) Application number: **91304217.2**

(22) Date of filing: **10.05.91**

(51) Int. Cl.⁵: **C12N 15/70, C12N 15/76,** // (C12N15/70, 15:16)

(30) Priority: **10.05.90 US 522352**

(43) Date of publication of application:
**13.11.91 Bulletin 91/46**

(84) Designated Contracting States:
**AT BE CH DE DK ES FR GB GR IT LI LU NL SE**

(71) Applicant: **ELI LILLY AND COMPANY**
**Lilly Corporate Center**
**Indianapolis Indiana 46285 (US)**

(72) Inventor: **Hershberger, Charles Lee**
**722 South 600 West**
**New Palestine, Indiana 46163 (US)**
Inventor: **Larson, Jeffrey Lynn**
**8210 Hoover Lane**
**Indianapolis, Indiana 46260 (US)**

(74) Representative: **Hudson, Christopher Mark et al**
**Erl Wood Manor**
**Windlesham Surrey GU20 6PH (GB)**

(54) **Heterologous promoter systems.**

(57) Provided are DNA sequences functional as a promoter in a heterologous host cell, said sequence derivable from an organism selected from the group consisting of Streptomyces griseofuscus, an actinophage, and mutants, derivatives, and variants thereof. Also provided are vectors and host cells containing the noted DNA sequences.

EP 0 456 507 A2

This invention relates to DNA sequences which are functional as promoters in a heterologous host cell, said sequence being derivable from an organsim selected from the group consisting of Streptomyces griseofuscus, an actinophage, and mutants, derivatives or variants thereof.

Most gene expression studies, for example those in which the host cell is E. coli, use homologous promoters, or mutants or hybrids of homologous promoters for transcription and subsequent translation of a desired structural gene. Often, mutant homologous promoters are generated for testing based partly on the assumption that the natural promoters may not provide a maximum level of gene expression. This assumption may be reasonable because environment and growth conditions for cultures in a laboratory setting or in production facilities are much different from the conditions found in nature. The nucleotide sequence and structure of promoters obtainable by natural selection, therefore, may not provide maximum levels of gene expression in the artificial conditions used to grow cultures in a laboratory or in a commercial setting. Consequently, improvements in fermentation capacities require one to constantly identify and develop experimental conditions that maximize the level of product accumulation.

Additionally, natural promoters may not provide maximum levels of gene expression because too much expression may be harmful to the host cells. Thus, natural selection actually may cause evolution of promoters that express optimum rather than maximum levels of gene product.

A number of experimental approaches have been used to improve naturally-occurring promoters. For example, researchers may mutate naturally-occurring promoters and screen for mutants that show high levels of expression. A second approach, analogous to evolution, is to synthesize oligonucleotides without regard to any knowledge of promoter sequences and to screen the oligonucleotides for promoter activity. A third approach is to identify a consensus sequence for a promoter and synthesize a related sequence which is not known to occur naturally.

Investigators have questioned whether promoters of one species would be able to express a protein in a foreign ("heterologous") genus or species. See, for example, Kovacevic et al., U.S. Patent No. 4,559,300 (Dec. 17, 1985), equivalent to European Publication, EP A 0 117 041 (published Aug. 29, 1984); and, Kuhstoss et al., U.S. Patent No. 4,766,066 (Aug. 23, 1988), equivalent to European Patent Publication, EP A 0 177 228 (Apr. 9, 1986). Unfortunately, very few such "heterologous" promoters have been found. Even so, most promoters express only at low levels, if at all, when inserted in a foreign host. In addition, the factors controlling promoter strength are poorly understood. Only a few promoters, especially those from Streptomycetes, are known to function in a heterologous host, for example from a different genus or species, such as E. coli. See, for example, the references, supra; and Forsman et al., Mol. Gen. Genet. 210, 23 (1987); Bibb et al., Mol. Gen. Genet. 187, 265 (1982); Jaurin et al., Gene 39, 191 (1985); Deng et al., Gene 43, 295 (1986). Furthermore, although many E. coli sequences can function as promoters in Streptomyces, very few Streptomyces sequences can act as promoters in E. coli. See, for example, Bibb et al., supra, Horinouchi et al., J. Bacteriol. 162, 406 (1985).

Until the present invention, however, it was not known whether a foreign ("heterologous") promoter would be stronger in a given species (for example, E. coli) than a naturally-occurring promoter from that same species. Particularly useful would be strong promoters from a non-homologous host which exhibit equal or greater expression in E. coli, the organism most commonly used in the recombinant DNA fermentation art and in commercial settings.

The present invention provides such DNA sequences. In particular, the present invention provides DNA sequences which are functional as promoters in a heterologous host cell, said sequence being derivable from an organism selected from the group consisting of Streptomyces griseofuscus, an actinophage, and mutants, derivatives and variants thereof. In a preferred embodiment, the actinophage is FP43, FP22, or a mutant, derivative or variant thereof. The invention also provides recombinant DNA vectors and host cells comprising the promoters of the invention. In a further embodiment, there is provided a method for expressing in E. coli a functional polypeptide which comprises

    (a) transforming an E. coli host cell with a recombinant DNA expression vector, said vector comprising:
        (1) a promoter, as defined above,
        (2) a translational activating sequence; and
        (3) a gene encoding a functional polypeptide; and
    (b) culturing the transformed E. coli cell under conditions suitable for expression of said polypeptide.
Further aspects of the invention are described in detail below.

Those skilled in the art will appreciate that the representative figures are drawn approximately to scale. The spacing of restriction sites on the map is not exact and actual restriction sites on the vector may vary somewhat from calculated distances. The maps do not provide an exhaustive listing of all the restriction sites on a given vector. These drawings are provided to aid the reader in better understanding the invention.

Figure 1 provides the restriction site and function map of plasmid pHJL35.

Figure 2 provides the restriction site and function map of generalized plasmid pHKY(HEPS).

Figure 3 provides the restriction site and function map of plasmid pHJL45.

Figure 4 provides the restriction site and function map of generalized plasmid pHKY(TN5).

Figure 5 provides the restriction site and function map of plasmid pHJL48.

Figure 6 provides the restriction site and function map of plasmid pHJL302.

Figure 7 provides the restriction site and function map of plasmid pHJL400.

Figure 8 provides the restriction site and function map of plasmid pHJL401.

Figure 9 provides the restriction site and function map of plasmid pHJL433.

Figure 10 provides the restriction site and function map of plasmid pHJL379.

Figure 11 provides the restriction site and function map of plasmid pHJL388.

Figure 12 provides the restriction site and function map of generalized plasmid pHJL(HEP/TN/302).

Figure 13 provides the restriction site and function map of generalized plasmid pHJL(HEP/TN/400).

Figure 14 provides the restriction site and function map of generalized plasmid pHJL(HEP/TN/401).

Figure 15 provides the restriction site and function map of generalized plasmid pHKY(PUC).

Figure 16 provides the restriction site and function map of plasmid pHJL52.

Figure 17 provides the restriction site and function map of generalized plasmid pHKY(HEP/hGH).

As noted above, the present invention provides a DNA sequence functional as a promoter in a heterologous host cell said sequence derivable from an organism selected from the group consisting of <u>Streptomyces griseofuscus</u>, an actinophage, and mutants, derivatives, and variants thereof. These promoters, which will be described in greater detail below, are referred to herein as "heterologous promoters". The nature of these heterologous promoters, is such that they are capable of promoting transcription in a host cell other than that in which they naturally are found or function, that is in a "heterologous host cell". Thus, a heterologous promoter derivable from an actinophage, for example, is a promoter capable of promoting transcription in a host cell other than an actinomycete which naturally serves as its host. In a preferred embodiment of the invention, the promoter, derived from <u>Streptomyces griseofuscus</u>, the actinophage, or a mutant, derivative or variant of either, is functional as a promoter in a <u>E. coli</u> host cell. Such a promoter is referred to as a heterologous <u>E. coli</u> promoter ("HEP").

Although, in the preferred embodiment, the heterologous promoters are functional in <u>E. coli</u>, the term "heterologous host cell" is meant to encompass other prokaryotic host cells in which the promoters of the invention function. Such a host cell may be, for example, other <u>Streptomyces</u> species, a <u>Bacillus</u>, <u>Pseudomonas</u>, <u>Agrobacterium</u>, <u>Staphlococcus</u>, <u>Streptococcus</u>, or <u>Serratia</u> cell. Using the protocols defined below, one can determine whether the promoters of the invention function in eukaryotic host cells such as fungi, for example, <u>Neurospora</u>, <u>Cephalosporium</u>, <u>Aspergillus</u>, <u>Penicillium</u>, and yeast; or cells susceptible to culture that are derived from tissue of multicellular organisms, including but not limited to a mammalian cell, murine mammalian cell, a human cell, an avian cell, an animal cell, a plant cell; and free living unicellular organisms, for example, algae or protozoans. Any such promoters functional in a eukaryotic host cell are meant to be encompassed by the present invention.

As used in the description and claims, these terms are defined as follow:

Recombinant DNA Cloning Vector—any autonomously replicating or integrating agent, including, but not limited to, plasmids, comprising a DNA molecule to which one or more additional DNA segments can be or have been added.

Recombinant DNA Expression Vector—any recombinant DNA cloning vector into which one or more transcriptional and/or translational activating sequence(s) have been incorporated.

Recombinant DNA vector—a recombinant DNA cloning or expression vector. Transcriptional Activating Sequence— any DNA sequence that directs or provides for the transcription of DNA into an mRNA transcript.

Translational Activating Sequence—any DNA sequence that provides for the translation of an mRNA transcript into a peptide or polypeptide.

<u>E. coli</u> Origin of Replication ("ori")— a DNA sequence that controls and allows for replication of a plasmid or other vector in E. <u>coli</u>.

<u>Streptomyces</u> Origin of Replication—a DNA sequence that controls and allows for replication of a plasmid or other vector in <u>Streptomyces.</u>

Promoter—a DNA sequence which promotes or provides for transcription of a DNA sequence to produce an mRNA transcript.

Actinophage—a bacteriophage which has as a natural host a microorganism which is an actinomycete, including members of the order Actinomycetales such as the genera <u>Actinomyces</u>, <u>Streptomyces</u>, <u>Nocardia</u>, and <u>Mycobacterium.</u>

Transformation—the introduction of DNA into a recipient host cell that changes the genotype and results in a change in the recipient cell.

Transfectant--a recipient host cell that has undergone transformation by phage DNA.

Transformant (transformed host cell)--a recipient host cell that has undergone transformation.

Sensitive Host Cell--a host cell that cannot grow in the presence of a given antibiotic without a DNA segment that provides resistance thereto.

Restriction Fragment--any linear DNA molecule generated by the action of one or more restriction enzymes.

Amp$^r$--the ampicillin-resistant phenotype.

Cm$^r$--the chloramphenicol-resistant phenotype.

Ts$^r$--the thiostrepton-resistant phenotype

cat--a gene encoding a chloramphenicol acetyl transferase.

MCS--a multiple cloning site fragment, also referred to as a "polylinker".

pro--a designation that a DNA sequence includes a promoter.

Tc$^r$--the tetracycline-resistant phenotype.

The preferred source of the actinophage derivable promoter is FP43, FP22, or a rapid lysis deletion mutant, FP22r4. FP43 and FP22, and the applicable host range of each, are described in Cox, K. et al., J. Bacteriology, 159 499 (1984). Also noted in Cox et al. are other actinophages which could be used as source DNA for the present invention. Other actinophage, however, may be used as a source of DNA to define heterologous promoters which fall within the scope of the invention. Thus, nothing herein is to be construed as limiting which particular actinophage is used as a starting material for purposes of the present invention. Table I provides an exemplary listing of actinophage which may be used as a source of DNA to be screened, using the methods provided, for heterologous promoter activity. Of course, one skilled in the art will recognize that other actinophage also will be suitable for this purpose.

## TABLE I

| Phage | Host | Reference |
|---|---|---|
| φC31 | *Streptomyces coelicolor* A3(2) | Chater et al., Gene, 15 249 (1981) |
| TG1, TG12 | *Streptomyces cattleya* *Streptomyces avermitilis* *Streptomyces virido-chromogenes* *Streptomyces garyphalus* | Foor et al., U.S. Pat. No. 4,460,689 |
| SH10 SH3 SH11 SH12 | *Streptomyces hygroscopicus* | Klaus et al., Mol. Gen. Genet. 172, 319 (1979) |
| 22653 | *Streptomyces griseus* | Mycologia, 45 209 (1953) |
| L1 | *Mycobacterium sp.* | Murohashi, K. Med. J., 34 1360 (1960) |
| BK3 | *Mycobacterium smegmatis* | Baess, Soil Acta Path. Microbiol. Scand. 75 331 (1967) |
| MC-2 | *Mycobacterium smegmatis* | Imaeda, "Host-Virus Relationships in Mycobacteria, Nocardia, and Actinomyces", Juhasz and Plummer, eds., pp. 189, (1970) C.C. Thomas, Publisher |

Likewise, natural or artificial mutants, variants or derivatives of any of the actinophage or Streptomyces griseofuscus used as DNA source strains are meant to be encompassed by the present invention. As one skilled in the art will recognize, artificial mutations, or variations can be obtained by treatment with various known mutagens such as ultra-violet rays, X-rays, high-frequency waves, radioactive rays and chemicals.

Further, mutations or derivatives of the parental DNA can be prepared using standard recombinant DNA techniques such as site-directed mutagenesis and deletion techniques. Such methods now are well-known to those skilled in the art. See, for example, Sambrook, J. et al., "Molecular Cloning: A Laboratory Manual", Second Edition, Cold Spring Harbor Laboratory Press, Sections 15 et seq. (1989).

In addition, although specific promoter probe vectors are described below, one skilled in the art will recognize that other such vectors can be prepared using the same or different reporter genes. Several such promoter probe vectors have been described. See, for example, Bibb, M. et al., Mol. Gen. Genet., 187 265 (1982); Ward, J.M. et al., Mol. Gen. Genet. 203, 468 (1986). To test for promoter activity in E. coli and/or Bacillus (e.g. Bacillus subtilis), the heterologous promoter containing fragments can be cloned into the promoter probe shuttle vectors as described in Sonenshein et al., U.S. Patent No. 4,725,535. For other promoter probe vectors for cloning in E. coli, see, for example, West et al., Gene 7, 271 (1979).

Further to construct shuttle vectors which can be used as promoter probes, one skilled in the art will recognize that the vector must contain an origin of replication ("replicon") which allows for replication in the desired host strain. For example, in the shuttle vectors of the present invention, the plasmid SCP2* origin of replication is used for vectors which are transformed into Streptomyces host cells. The pBR322 or a pUC plasmid origin of replication is used herein for those vectors which are transformed into E. coli host cell. Table II provides an exemplary listing of Streptomyces plasmids from which other Streptomyces replicons can be obtained. All or part of these plasmids can be used to construct the desired promoter probe vector as long as the replicon function is not disturbed.

## Table II

### Streptomyces Plasmids

| Plasmid | Host | Accession No. |
|---------|------|---------------|
| SCP2 | Streptomyces coelicolor A3 (2) | NRRL[‡] 15042 |
| SCP2* | Streptomyces coelicolor M110 | NRRL 15041 |
| pEL7 | Streptomyces ambofaciens pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB[**] 11417 |
| pNM100 | Streptomyces virginiae | NRRL 15156 |
| pEL103 | Strptomyces granuloruber A39912.13/pEL103 | NRRL 12549 |

[‡]Agricultural Research Culture Collection (NRRL), 1815 North University Street, Peoria, Illinois 61604, United States of America

**National Collection of Industrial Bacteria (NCIB), Torry Research Station, Post Office Box 31, 135 Abbey Road, Aberdeen AB98DG, Scotland, United Kingdom

If one wants to create promoter probes functional in other heterologous hosts, for example, Bacillus, the reader is referred to U.S. Patent No. 4,783,405. This patent discloses expression vectors which are functional in Bacillus and which contain a Bacillus origin of replication. Upon removal of the veg promoter portion of these vectors and insertion of a suitable reporter gene, these vectors could be used as promoter probe vectors for heterologous promoters which are functional in a Bacillus host strain.

Because amplification and manipulations of plasmids generally is faster in E. coli than other host strains, and because E. coli is the most preferred heterologous host for the promoters of the invention, it is desirable to add to the vectors disclosed DNA sequences which allow for replication in E. coli. Thus, the addition of a functional replicon-containing and/or antibiotic resistance conferring restriction fragments from E. coli plasmids, for example, pBR322, pACY184, pBR325, pBR328, or the like, is highly advantageous and adds to the general utility of the vectors of the invention.

Likewise, one skilled in the,art will recognize that for probes useful in other hosts, a vector can be constructed which comprises an origin of replication and a reporter gene which function in the desired host cell. For selection purposes, one or more antibiotic resistance-conferring fragments may be contained on the desired vector.

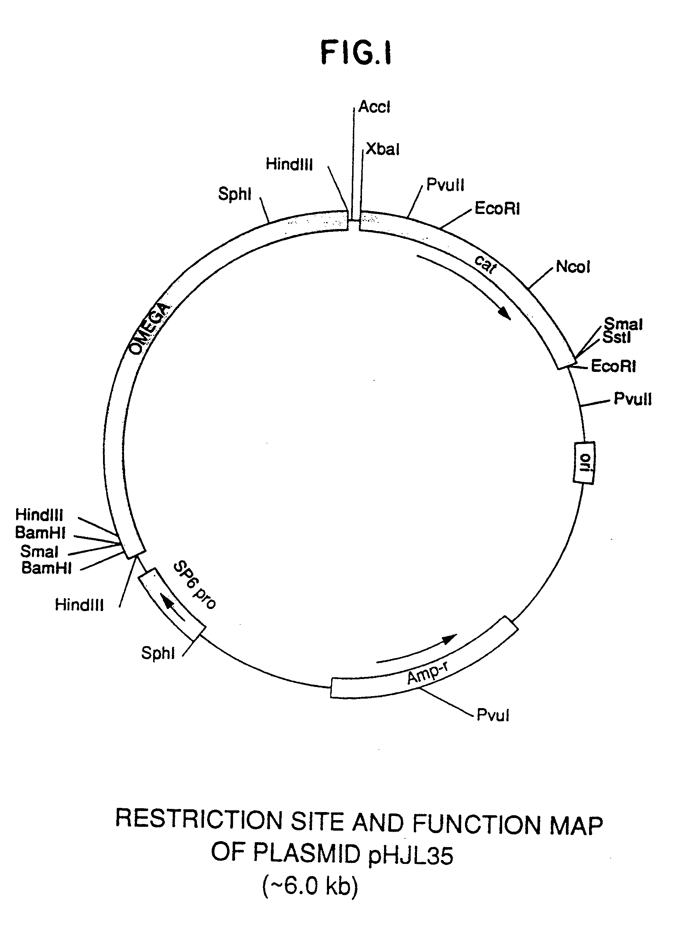

To select the heterologous promoters of the invention, genomic DNA from Streptomyces griseofuscus, or an actinophage, in particular, FP43 or an FP22 rapid lysis mutant, FP22r4, was screened for promoter activity in E. coli using promoter probe vectors which contained a DNA sequence encoding chloramphenicol acetyltransferase (cat) as the reporter gene. To construct this promoter probe vector, a promoterless cat gene was cloned into Riboprobe™ vector, pSP64, available from Promega Biotech (2800 S. Fish Hatchery Rd., Madison Wisconsin). In particular, a promoterless cat structural gene, which contained its natural ribosomal binding site ("rbs"), was obtained on a ~ 1.0 kb Sau3A restriction fragment and cloned into the BamHI site of pSP64. This method produces plasmids which contain the cat gene in both possible orientations. One of these constructions, plasmid pHPR66, had the cat structural gene situated such that the HindIII, PstI, SalI (AccI and HincII also) and XbaI sites are proximal to the cat ribosomal binding site. Plasmid pHPR66 confers high level (>25 μg/ml) resistance to chloramphenicol, presumably because of read-through transcription from promoters present elsewhere on the plasmid. To prevent read-through expression of the cat gene, the "omega" fragment (See Prentki et al., Gene, 29, 303 (1984)) is inserted into the vector. The omega fragment contains a gene conferring resistance to spectinomycin and streptomycin, the bacteriophage T4 transcription stop sequence from gene 32 and 3 stop codons, each in a different reading phase. Cleavage of the omega fragment with HindIII followed by ligation into HindIII cleaved plasmid pEPR66 produced plasmid pHJL35. Plasmid pHJL35, transformed into E. coli K12 MM294, has been deposited (April 16, 1990) in accordance with the terms of the Budapest Treaty at the Northern Regional Research Laboratory ("NRRL"), Peoria, Illinois. A sample of this transformed host is available from the NRRL under the accession number NRRL B-18645. A restriction site and function map of plasmid pHJL35 is provided in Figure 1.

Plasmid pHJL35 contains at the ligation junction farthest from the cat structural gene a small duplication of the HindIII-BamHI-SmaI portion of the omega fragment which had been cleaved prior to the ligation. The insertion of the omega fragment did not disrupt the polylinker sites proximal to the cat structural gene. Plasmid pHJL35 confers resistance to streptomycin or spectinomycin and ampicillin in E. coli but fails to provide resistance to chloramphenicol at levels of >10 μg/ml.

Plasmid pHJL35 is useful as a promoter probe vector because it contains a unique AccI site. Cleavage with AccI leaves a 5'-two base pair CG overhang. Upon cleavage with AccI, this site can be ligated to a large number of restriction fragments which result from the cleavage with a variety of enzymes which leave a 5'-CG overhang. For example, the following enzymes produce compatible 5'-CG overhangs: ClaI, AsuII, AhaII, NarI, MspI, HpaII, HinPI, and TaqI. Thus, the AccI site of pHJL35 is useful for cloning a variety of restriction fragments.

To determine whether a fragment contains a heterologous promoter of the invention, one skilled in the art need only clone into the unique AccI site of plasmid pEJL35, a compatibly restricted DNA fragment from the desired organism. In particular, DNA from the desired actinophage or Streptomyces griseofuscus may be cleaved with any restriction enzyme, such as those noted above, which provide ends compatible to the AccI-cleaved plasmid pHJL35. These fragments then are cloned into the AccI site of plasmid pHJL35. Transformation into the desired heterologous host followed by selection on media containing chloramphenicol will allow one skilled in the art to determine which colonies are transformed with plasmids containing a heterologous promoter of the invention. Alternately, one skilled in the art could construct appropriate linker sequences to allow cloning into the AccI site of plasmid pHJL35.

In a preferred embodiment of the invention, three organisms were used as the source of heterologous promoters of the invention. As previously noted, genomic DNA from Streptomyces griseofuscus, an actinophage, FP43, and a rapid lysis deletion mutant of actinophage FP22, FP22r4, were used as sources of the DNA to be cloned into the AccI site of plasmid pHJL35. Streptomyces griseofuscus is available from the American Type Culture Collection ("ATCC"), 12301 Parklawn Drive, Rockville, Maryland 20852 under the accession number ATCC 23916. Actinophage FP43 is deposited in the NRRL (February 26, 1987) as a transfectant in Streptomyces griseofuscus and is available under the accession number NRRL 18184. Actinophages FP22 and FP22r4 have been deposited (April 25, 1990) under the terms of the Budapest Treaty and are available from the American Type Culture Collection ("ATCC"), Rockville, Maryland 20852, under accession numbers ATCC 40798 and ATCC 40799, respectively.

In a preferred embodiment of the invention, the genomic DNA from the desired organism is cleaved with a restriction enzyme compatible with the terminii of AccI-cleaved plasmid pHJL35. A particularly preferred enzyme for this purpose is Taql. The AccI-cleaved plasmid pHJL35 is prepared and ligated to the Taql cleaved DNA.

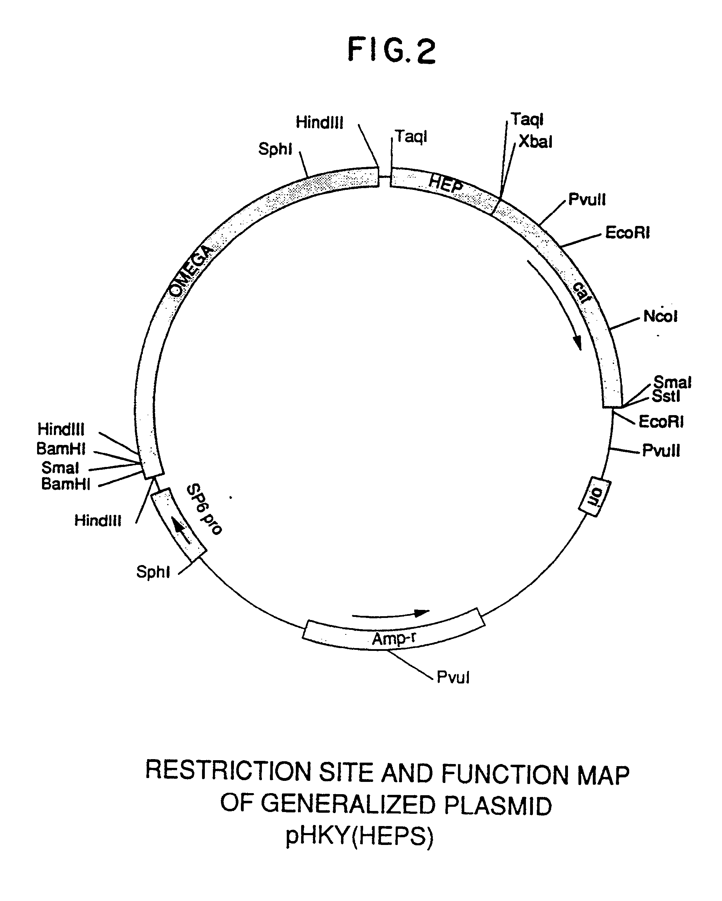

After ligation, the resulting plasmids are transformed into the desired host strain. In an especially preferred embodiment, for example, one using plasmid pHJL35 as a promoter probe vector, the plasmid containing the Streptomyces or actinophage DNA is transformed into E. coli. Upon transformation, the host cell is grown on a culture medium containing ampicillin and subsequently tested for growth on medium containing streptomycin and chloramphenicol. Those colonies which grow under these selection conditions are considered to contain a heterologous promoter of the present invention. For ease of reference, those plasmids derived in this manner and which contain the promoters of the invention have been designated by the general plasmid name pHKY(HEPS). A restriction site and function map of this generalized plasmid is shown in Figure 2. Using the methods just described, approximately 165, 400, and 1640 chloramphenicol resistant clones were isolated using DNA from Streptomyces griseofuscus, actinophage FP43 and actinophage FP22r4, respectively.

In an additionally preferred embodiment of the invention, the plasmids having inserts from FP22r4 provide the highest level of chloramphenicol resistance. Of the FP22r4 heterologous promoter containing plasmids, nine showed particularly high levels of resistance to chloramphenicol (>1,000 µg/ml). These nine plasmid pHJL35 derivatives have been designated plasmids pHKY23, pHKY173, pHKY177, pHKY179, pHKY180, pHKY183b, pHKY183, pHKY184 and pHKY185. The promoter portion (designated "HEP" in Figure 2) of these plasmids can be excised and sequenced using standard sequencing techniques. The following represents the heterologous promoter containing DNA sequence of an Xbal or HindIII restriction fragment of pHKY173:

```
  1   TCTAGAGTCG AACAATTCTA TACATTTTTC CACCACGCTG TCTAATGCCA

 51   TTAGCCTCGT TAACGAAGCG AACATCCGTC ACTACATAAT TCCTATTCAT

101   ATGCATGGAG TTGAATGTGG CATCTGCCCA AATTTCATTT CCGAGAATAC

151   CGCGAACACA GTTAGTACCA ACTACTTGCA TCCATCTTCT GATATCCTGA

201   GCCCAAATTG TATCTTTGTA CCCATTCCAT CCGTACCTAT CGAGACGCGG

251   AGCCAAACCT ACGTCGACCT GCAGCCCAAG CTT
```

This sequence contains a potential -10 and -35 E. coli promoter consensus sequence at positions 20 to 15 and 44 to 39, respectively. Further, this sequence is believed to contain a major transcript start signal at base 10 and minor transcript start signals at base 8 and base 9. In this case and in the sequences described below, transcription proceeds from right to left relative to the printed sequence.

A promoter containing Xbal or HindIII restriction fragment of plasmid pHKY177 has the following sequence:

```
  1   TCTAGAGTCG AACAATTCTA TACATTTTTC CACCACGCTG TCTAATGCCA

 51   TTAGCCTCGT TAACGAAGCG AACATCCGTC ACTACATAAT TCCTATTCAT

101   ATGCATGGAG TTGAATGTGG CATCTGCCCA AATTTCATTT CCGAGAATAC

151   CGCGAACACA GTTAGTACCA ACTACTTGCA TCCATCTTCT GATATCCTGA

201   GCCCAAATTG TATCTTTGTA CCCATTCCAT CCGTACCTAT CGATTACATC

251   CCGCAAATTT GCAATCATTC CATTACAGTT TAGTGGGGGA TTCATCTCAT

301   ACATGAATTC TCTCAATTTG TCAGCGAAGG CAATACGGCG AAAGCCGATG

351   CTCTCCAGAG CATCGGCTGC CGAATCCTTA CCTGACCTAG CGTATCCAGC

401   CAAGCCGATA ATCATTTTAC ATATACCTCA AAATCTTGTA TGCCTCTCGA

451   CCTGCAGCCC AAGCTT
```

This sequence has potential -10 and -35 E. coli consensus sequences at positions 20 to 15 and 44 to 39, respectively. Further, this sequence may contain a major transcript start at base 10 and minor transcript start at base 8 and base 9. One skilled in the art will note that the first 243 bases of the promoter containing fragments for plasmids pHKY173 and pHKY177 are identical. This identity reflects independent isolation of the same heterologous promoter. This independent isolation of the same heterologous promoter verifies the specificity of the protocol to isolate strong promoters.

The following is the sequence of the first 220 bases of an Xbal/HindIII fragment of pHKY179: Within the sequence is believed to be a -10 and -35 E. coli promoter consensus sequence located at bases 189 to 184 and 212 to 207, respectively.

```
  1   TCTAGAGTCG ACCTTGGCTA CGTCCTCCCA ATTAACGCCA TCCTCAGCGA

 51   AAGCAGCAAT AATTACAGCC TTCGTGTCAC TTGGCTTTAC ATCTACGCCG

101   TAATCGTCGG CAATCTTAAG AAGGTCTTCC TTCTTTAGTG TATCAAAACT

151   CATGGTATTT GGTATTCCTC CTATTGTGTT TTCATTATAG CAGACTGTTC

201   GTTCAACGAC AAAACCCCCG CT
```

This sequence may contain a major transcript start at base 176.

The following is a heterologous promoter containing sequence from plasmid pHKY180 sequenced downstream from its Xbal site:

```
  1  TCTAGAGTCG AGTCCTGTCG GGCGTACTTT ATCGTTAATT ACAAAGGGGA

 51  ATGTAATGGG TTACACGCGA GTCCGCAAGG GTGACAATGT TTTTGTCCAG

101  AAGGGTCCGG ACAA
```

This sequence may contain an initiation of transcription site at base 43 and a potential -10 and -35 E. coli consensus promoter sequence located at bases 55 to 50 and 78 to 73, respectively.

The following is the promoter containing sequence of the 195 base pair XbaI-HindIII fragment of plasmid pHKY183:

```
  1  TCTAGAGTCG AGTCCTGTCG GGCGTACTTT ATCGTTAATT ACAAAGGGGA

 51  ATGTAATGGG TTACACGCGA GTCCGCAAGG GTGACAATGT TTTTGTCCAG

101  AAGGGTCCGG ACAAGGGCAA GAAGTTGAAG GTCACGGAAG AGGGCAAGGG

151  TATGAACCGG GATTACATCA AGTTGTCGAC CTGCAGCCCA AGCTT
```

One skilled in the art will recognize the sequence identity for the first 114 bases of the promoter containing fragment of plasmids pHKY180 and pHKY183. This identity probably reflects separate isolation of the same heterologous promoter.

This fragment may contain a potential -10 E. coli promoter consensus sequence on the complementary strand at positions 38 to 43 or 162 to 167. Because the consensus sequence on the complementary strand does not promote transcription, one cannot predict from the sequence alone which consensus sequences will serve as strong promoters.

The following is a heterologous promoter containing sequence obtained from plasmid pHKY183b:

```
  1  TCTAGAGTCG ACAAACAATC TCAACAAGGT TTGACTCTTC CTGGCGATGA

 51  ACCGCAAAGG CCATGTCAGC ATCGTATTCA ATTGCCTTGG ACCATGCAAC

101  CTGACTCATC ATCGGAGGAG AATCACGGTC GGAAAGGTCT GACTGAGTTG

151  CAGCCGTAAT ATCAATCAGC GGGATATTGT TTGTAACCGC AAG
```

This fragment contains a potential -10 E. coli consensus sequence at positions 76 and 71 but it does not contain a -35 sequence separated by 16 to 19 base pairs from the -10 consensus sequence. This sequence may contain an initiation of transcription site at base 65. Also it may contain initiation of transcription sites at base 58 and base 35. These latter two initiation of transcription sites do not have a properly spaced nearby consensus -10 and -35 sequence. Thus, as noted earlier, it is not possible to predict correctly, by sequence alone, those sequences which may serve as promoters.

The following is a sequence, starting from the XbaI site, from plasmid pHKY184 which contains a heterologous promoter of the invention.

```
  1   TCTAGAGTCG ACACCAAGAC GCTCAGCGTT CTTGTTCACT GCATCAGGTG

 51   CCAACCATAC CTTGTCGTTG ACCATCATGT CAAACGCCCG GAATTCCAGA

101   CGCTCA
```

This sequence may contain a potential -10 and -35 E. coli consensus sequence located between bases 59 to 54 and 83 to 78, respectively. In addition, this sequence may contain initiation of transcription sites at base 47 and base 46.

The following represents a heterologous promoter containing sequence of the invention. In particular, the sequence below is a XbaI-TaqI restriction fragment of plasmid pHKY185:

```
  1   TCTAGAGTCG AACGCAGGAA TGCTCAGATA CTCGTAGAAG TAATGCCTCT

 51   CATCTCCTCG CTTAGGATTG TCCACAAAAG TGGCCGCTCC AATTGCATTC

101   AGAGACTTGA GAACCCTGAT ATTTAGCCCA TTACCCTTCT CCATAACCTT

151   TGCCTCTAGG GCAGCGTAAT TCTCGTAAGG TCGTGAGTCA ATCAACTTCT

201   TTCCGAGATT ATCCGAAATG AACTTGATGT TTGCCAGTCC GAATCGA
```

Potential transcription start sites may be located at positions 155, 47, 30, 29 and 13 of the sequence. None of these transcription start sites contains a consensus -10 sequence separated by 7 to 9 base pairs from the transcription start site. Therefore, none of the transcription start sites are predicted from the sequence alone. In addition, a -10 and a -35 E. coli-like consensus promoter sequence may be located at positions 174 to 169 and 195 to 190, respectively. In addition, this sequence may contain a promoter consensus sequence at bases 213 to 208 and 237 to 232, respectively. One skilled in the art will recognize that the last consensus sequence does not exhibit a nearby transcription initiation site. Therefore, it is not possible, from sequence alone, to say which consensus E. coli-like promoter sequences actually function as a promoter.

Thus, in an especially preferred embodiment of the invention, there is provided a heterologous promoter sequence contained within any of the DNA sequences noted above. As noted before, the features which determine strong promoters cannot be determined from the sequences alone and, therefore, are not predictable. Some of the identified E. coli-like consensus sequences observed do not promote high level expression and some of the transcription initiation sites do not contain properly positioned nearby E. coli promoter consensus sequences.

Having shown that the promoters of the invention are good heterologous promoters, one skilled in the art may desire to show that these are active promoters in their natural host. In so doing, one skilled in the art may be able to locate repressor or control elements for the heterologous promoters. In addition to examining transcription start sites, a vector system is needed which is capable of operating in either the heterologous or natural host.

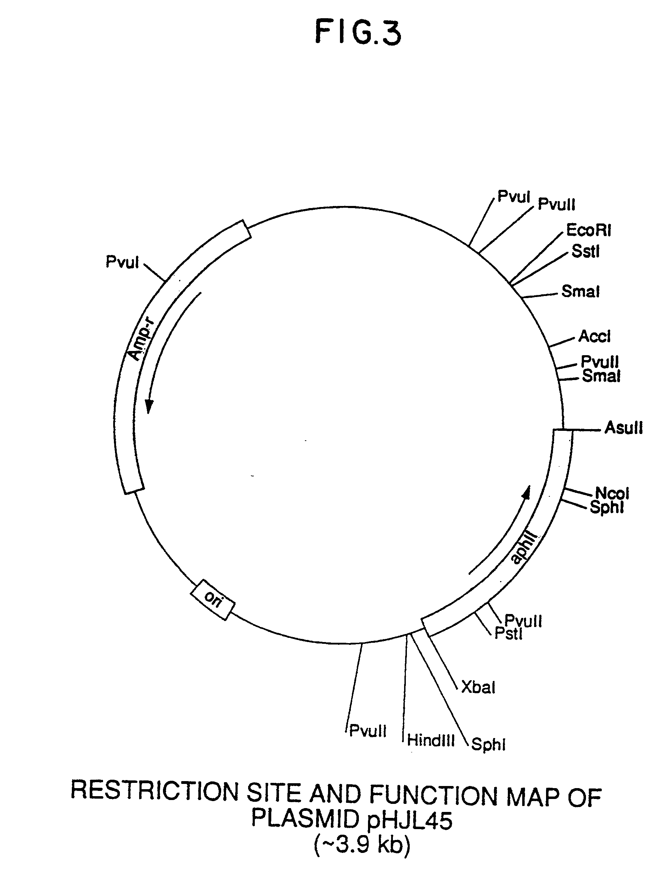

A particularly useful system for use in the preferred embodiment involves the use of the aphII gene from Tn5. This gene has been shown to be a good reporter gene in both E. coli and Streptomyces. The source of the Tn5 aphII gene was plasmid pIJ486 (see Bibb et al., Mol. Gen. Genet. 203 468 (1986)). An ~1.1 kb BclI restriction fragment from this plasmid is ligated into plasmid pUC19 (available commercially, for example, from New England BioLabs, Inc., Beverly, Mass. 01915), at its BamHI site (located within the polylinker sequence of that plasmid), to form plasmid pHJL45. Because this plasmid contains a promoterless aphII gene, the vector is useful in the construction of other promoter probe vectors which are functional in both E. coli and Streptomyces. Plasmid pHJL45 has been tranformed into E. coli K12 JM109 and then deposited (April 16, 1990) at the NRRL in accordance with the terms of the Budapest Treaty. A sample of this transformed host is available under the accession number NRRL B-18646. A restriction site and function map of plasmid pHJL45 is shown in Figure 3.

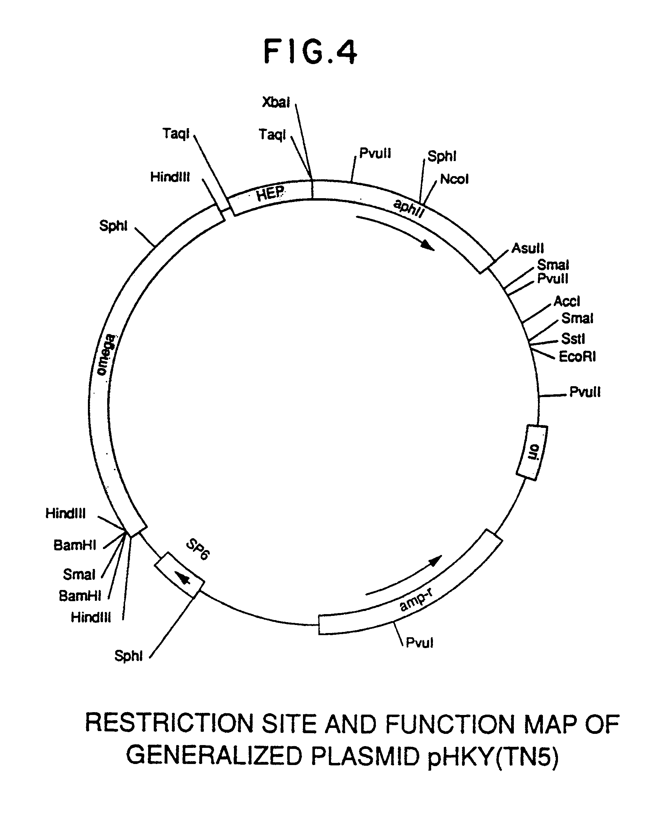

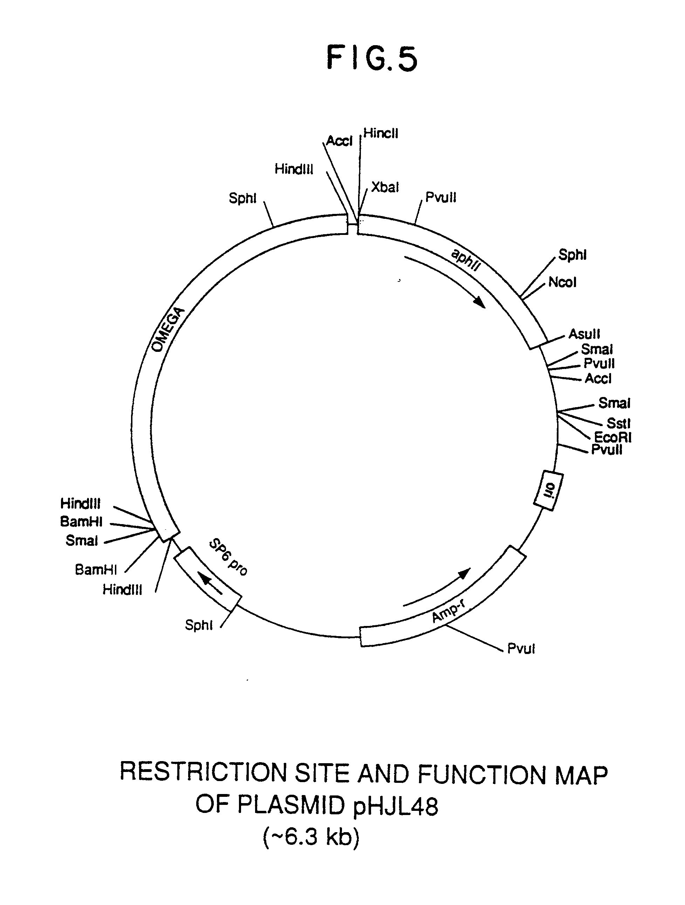

Plasmid pHJL45 is constructed such that the promoterless aphll gene is downstream from the Xbal site in the polylinker portion of the vector derived from plasmid pUC19. Thus, one skilled in the art using recognized restriction and cloning techniques, can replace the cat gene from a pHKY(HEPS) vector with the aphll gene from plasmid pHJL45. Generally, this may be accomplished, for example, by cleaving both the pHKY(HEPS) vector and plasmid pHJL45 with Xbal and Sstl restriction enzymes. Purification of the aphll containing fragment from plasmid pHJL45 and the large heterologous promoter containing fragment from plasmid pHKY(HEPS), followed by ligation of the fragments, will result in a series of new generalized plasmids designated pHKY(TN5). A restriction site and function map of generalized plasmids pHKY(TN5) is shown in Figure 4. Transformation into the desired host cell and selection on media containing ampicillin, and subsequent testing on media containing streptomycin and kanamycin provides those clones which contain the heterologous promoter sequence and the aphll gene. If a promoterless construct analogous to these vectors is desired, one skilled in the art will appreciate that the same procedure outlined above can be used to remove the cat gene from plasmid pHJL35, described above, and followed by insertion of the aphll gene of plasmid pHJL45 to produce exemplary new plasmid pHJL48. Generally, this scheme will work except in those circumstances in which the heterologous promoter sequence contains an internal restriction site which is the same as one of the restriction enzymes used to prepare the aphll-containing vector. Thus, in such a circumstance, alternate restriction enzymes, possibly in combination with a linker sequence, may be used to prepare the desired aphll-containing vector. A restriction site and function map of the promoterless aphll promoter probe, plasmid pHJL48, is shown in Figure 5.

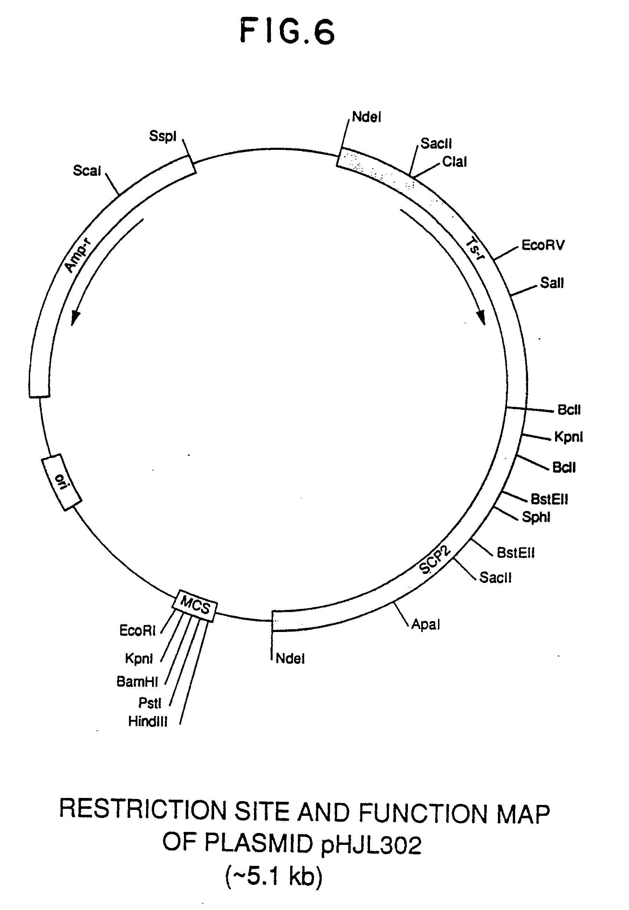

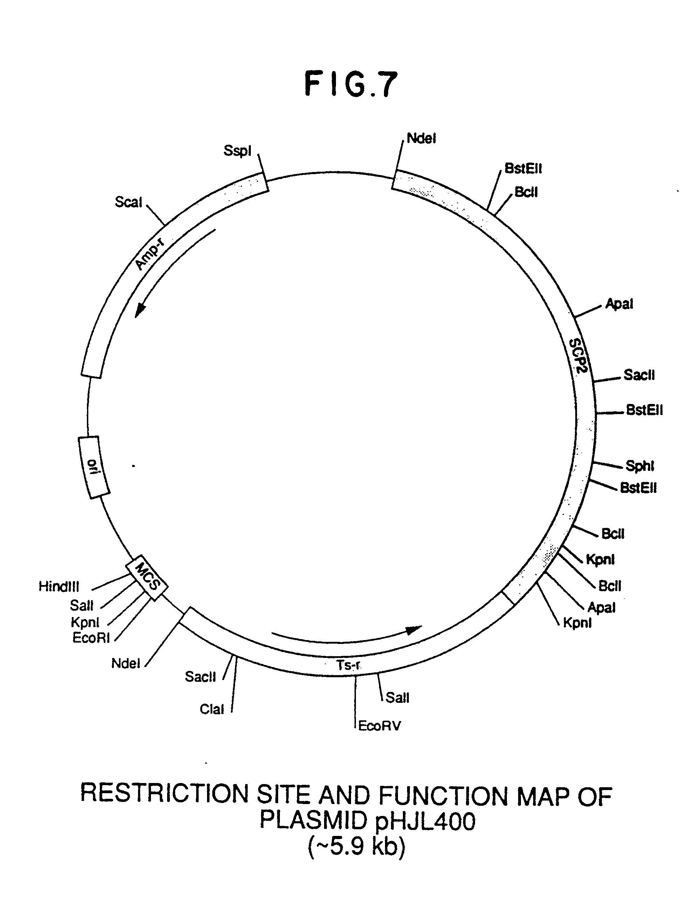

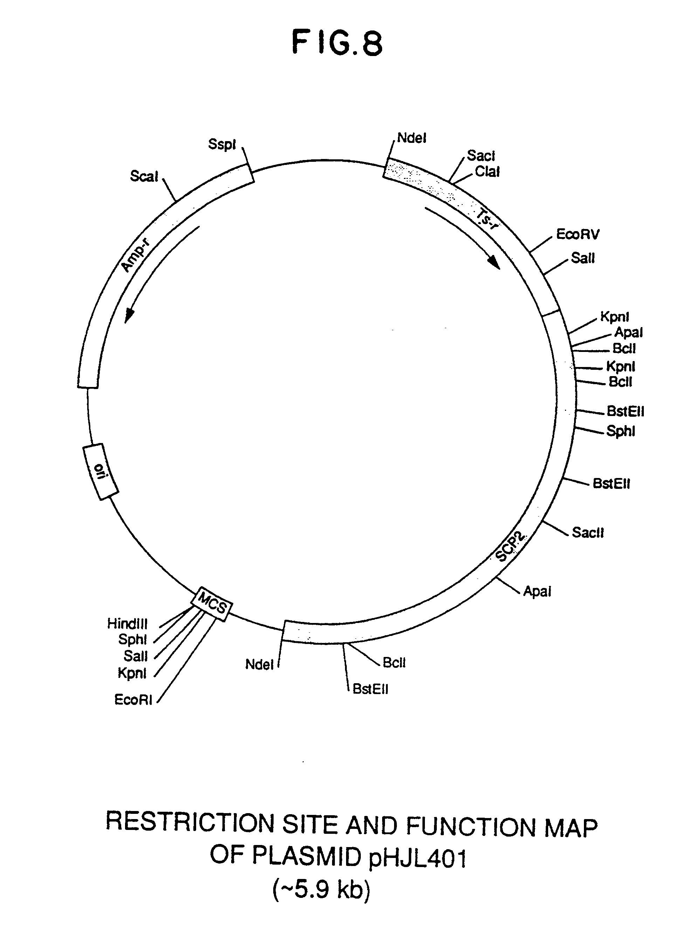

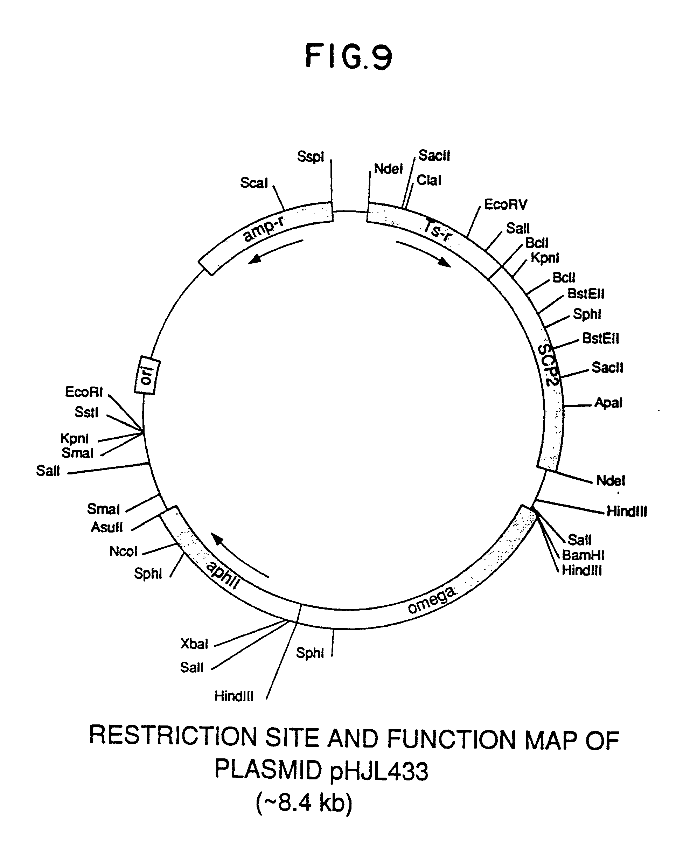

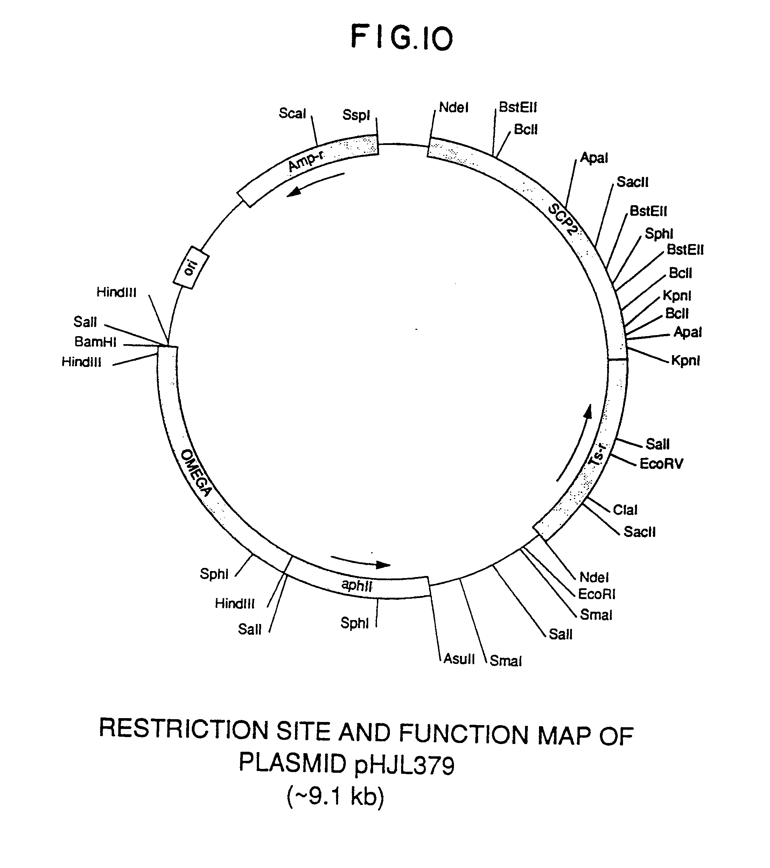

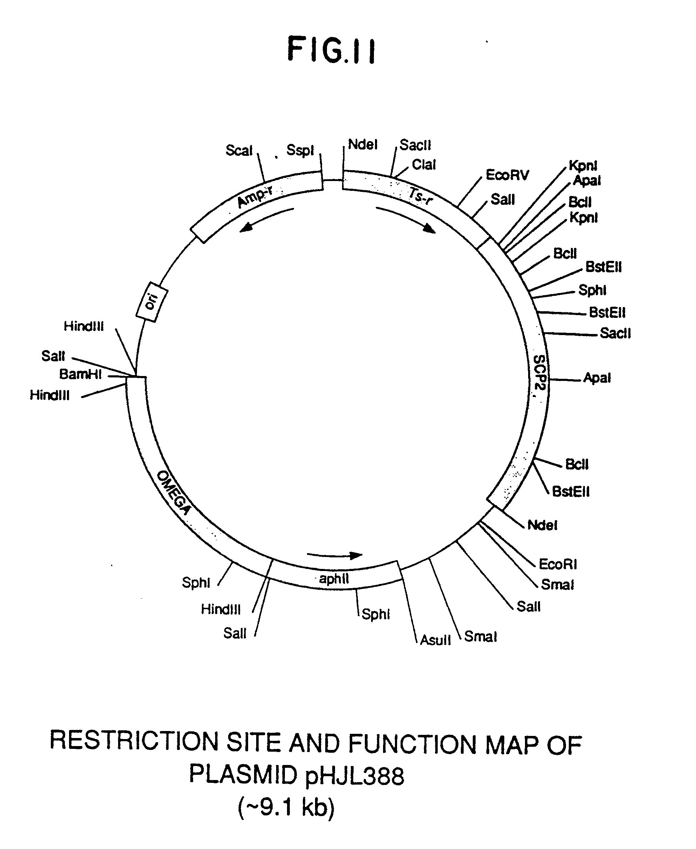

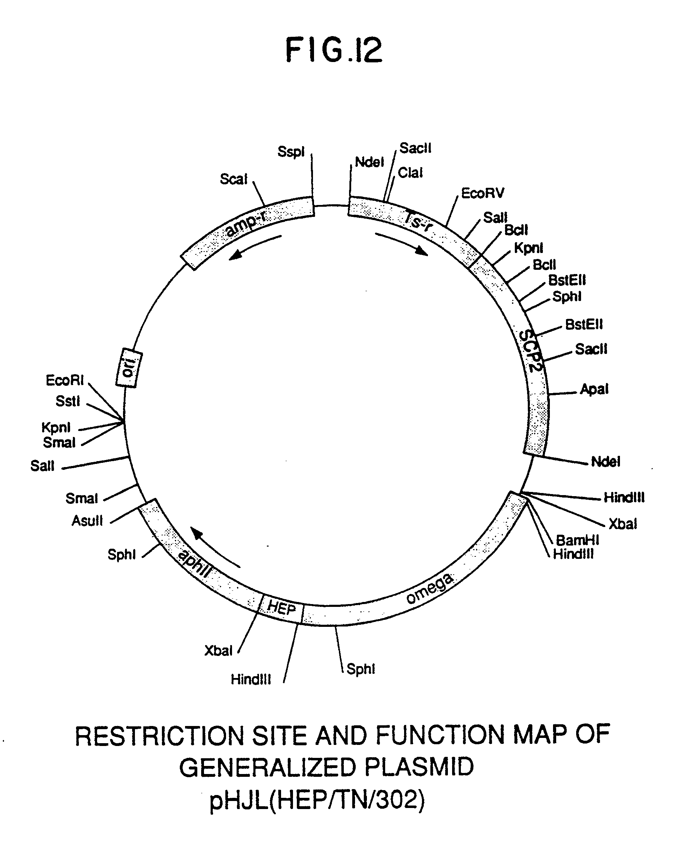

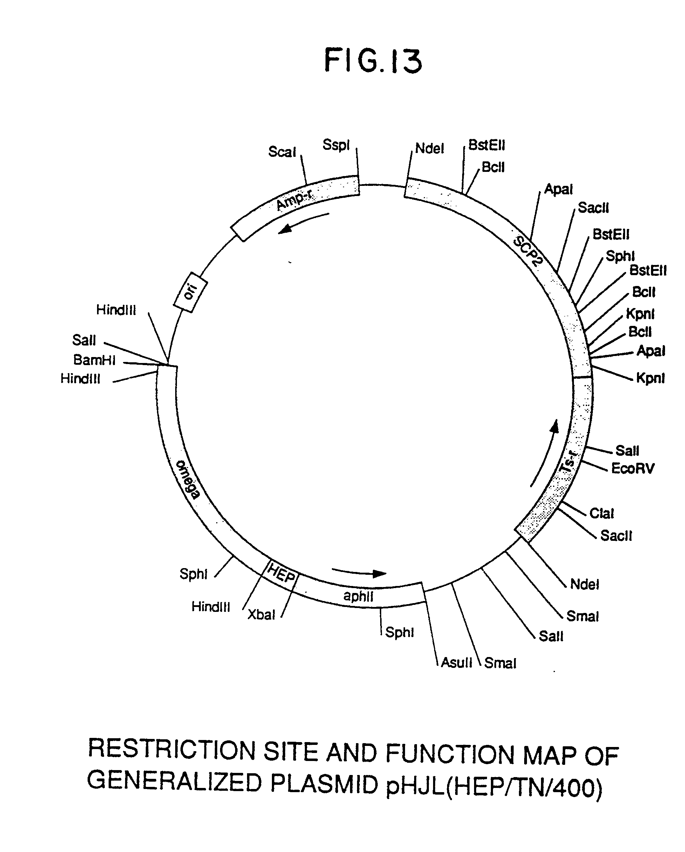

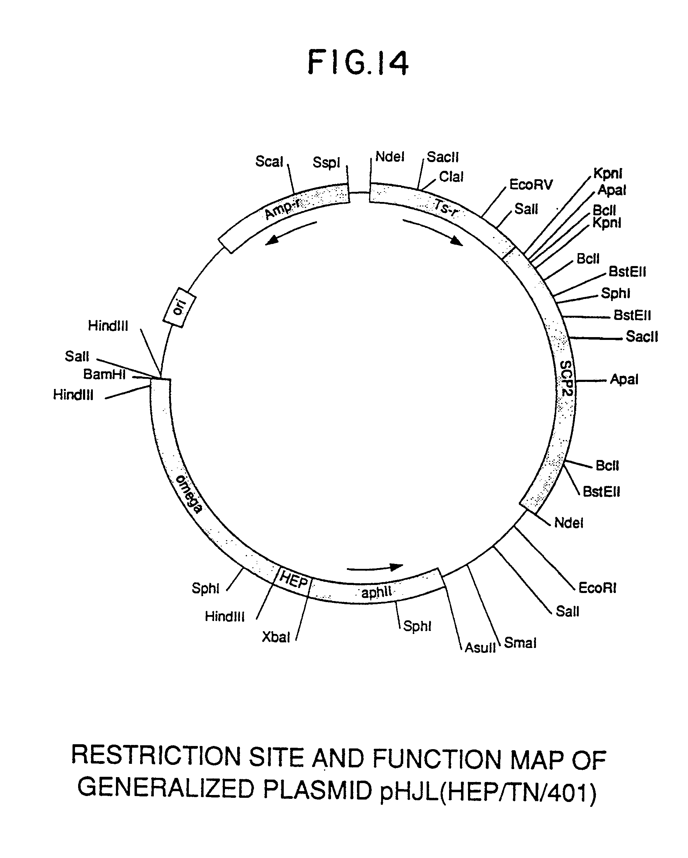

To demonstrate the strength of the preferred heterologous promoters of the invention in both Streptomyces and E. coli, the omega/heterologous promoter/Tn5 fragment expression system must be placed on a shuttle vector. Particularly useful shuttle vectors for this purpose are those prepared by inserting the SCP2* replicon into plasmids pUC19 and pUC18. For example, plasmid pHJL302, transformed into E. coli K12 JM109, has been deposited (April 16, 1990) under the terms of the Budapest Treaty at the NRRL and is available under the accession number NRRL B-18648. Plasmid pHJL302 is an ultra high copy shuttle vector useful for transformation of Streptomyces, in particular Streptomyces lividans. A restriction site and function map of plasmid pHJL302 is provided in Figure 6. This vector is constructed from an ultra high copy Streptomyces plasmid and pUC18. Additional shuttle vectors useful for this purpose are plasmids pHJL400 and plasmid pHJL401. The restriction site and function map for these plasmids is shown in Figures 7 and 8, respectively. These latter plasmids have been transformed into E. coli K12 JM109 and deposited at the NRRL under the terms of the Budapest Treaty and are available under the accession numbers NRRL B-18650 (April 27, 1990) and NRRL B-18217 (May 7, 1987), respectively. Plasmids pHJL400 and pHJL401 are moderate copy number Streptomyces vectors which are derived from a moderate copy number Streptomyces plasmid and pUC19. Plasmids pHJL302, pHJL400 and pHJL401 retain the multiple cloning site ("polylinker") of their plasmid pUC18 or pUC19 parent. Each of these plasmids can be cleaved with restriction enzymes BamHI and Sstl. The omega/heterologous promoter/Tn5 (aphll) fragment may be removed by treatment of the desired pHKY(TN5) plasmid with one of these restriction enzymes followed by purification of the desired fragments. Upon ligation of this fragment into any of similarly digested pHJL302, pHJL400 or pHJL401, the resulting plasmid can be transformed into an E. coli strain, for example, E. coli K12 JM109. E. coli K12 JM109, a well-known host, is commercially available from several sources, for example, Clontech Laboratories, Inc., Palo Alto, CA 94303 and Stratagene Cloning Systems, La Jolla, CA 92037. The BamHI site on plasmids pHJL302, pHJL400 and pHJL401 resides within a polylinker that itself forms a part of the DNA sequence encoding the lacZ α-fragment. Expression of the lacZ α-fragment in an E. coli ΔM15, or similar mutant, such as JM109, restores the mutant's ability to produce a functional β-galactosidase enzyme. Thus, plasmids pHJL302, pHJL400 or pHJL401 can restore. β-galactosidase activity to an E. coli ΔM15 mutant. However, insertion of DNA into a restriction site of the polylinker on these plasmids, as occurs in the construction of the plasmids described below, disrupts the lacZ α-fragment coding sequence and concomitantly destroys the ability of the pHJL302, pHJL400 or pHJL401 derivative to complement a ΔM15-type mutation. β-Galactosidase can hydrolyze X-Gal, which is 5-bromo-4-chloro-3-indolyl-β-D-galactosidase, a colorless compound, to an indigo-colored product and thus allows for a convenient screening method for discriminating between transformants containing starting plasmids pHJL302, pHJL400 or pHJL401 or a modified plasmid, containing an insert in the polylinker portion of the plasmid. Thus, transformants which are white on X-Gal medium and which are resistant to ampicillin, kanamycin and streptomycin are selected and verified by mini-prep techniques to be the desired construction. The resulting shuttle vector then can be isolated and transformed into Streptomyces lividans, or other suitable host, and the level of kanamycin resistance can be studied. For control purposes, corresponding promoterless constructions can be made in a manner, analogous to that described above, using pHJL48, described above, as the source of the omega/Tn5 (aphll) fragment. These promoterless constructs are designated as plasmids pHJL433, pHJL379 and pHJL388 and are derived from pHJL302, pHJL400 and pHJL401, respectively. A restriction site and function map of each of these plasmids is given in Figures 9, 10, and 11, respectively. The desired heterologous promoter-containing shuttle vectors prepared as described above, have been designated as generalized plasmids pHJL(HEP/TN/302), pHJL(HEP/TN/400) or pHJL(HEP/TN/401) depending on whether pHJL302, pHJL400, or pHJL401, respectively, is the source of the Streptomyces replicon containing fragment. A restriction site and function map of each of these plasmids is shown in Figures 12, 13, and 14, respectively.

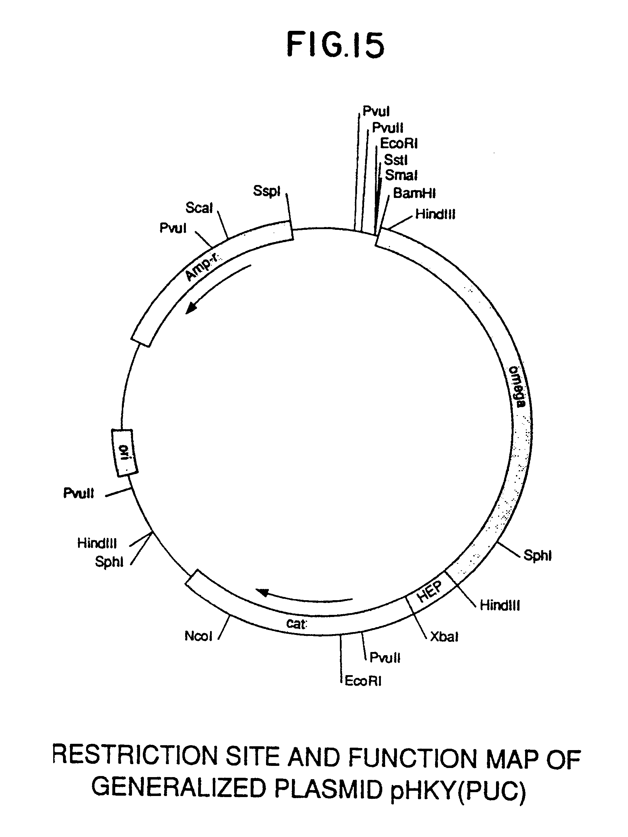

The heterologous promoters of the present invention can be used to express a desired polypeptide. To show this, several of the promoters of the invention were inserted in a recombinant DNA expression vector which comprised a promoterless synthetic human growth hormone encoding sequence which has been designated Met-Asp-hGH. An amino acid sequence and the corresponding DNA encoding region of Met-Asp-hGH is described in U.S. Patent No. 4,874,703 (issued October 17, 1989). To construct Met-Asp-hGH expression plasmids which contain the heterologous promoters of the invention, the omega/heterologous promoter/cat fragment from plasmid pHKY(HEPS) is inserted into plasmid pUC19. In particular, a pHKY(HEPS) plasmid is treated with BamHI and SmaI restriction endonucleases, followed by separation of the desired fragments. Plasmid pUC19 then is cleaved with restriction enzymes BamHI and HincII. The BamHI/SmaI fragment from pHKY(HEPS) and the BamHI/HincII fragment from plasmid pUC19 are ligated to form generalized plasmids pHKY(PUC). The restriction site and function map of plasmids pHKY(PUC) is shown in Figure 15. These plasmids are especially useful because they have an EcoRI and SstI site upstream from the omega/heterologous promoter fragment. Thus, digestion of plasmids pHKY(PUC) with EcoRI and XbaI will remove the omega/heterologous promoter fragment for insertion in the desired expression vector. Alternatively, if plasmid pHKY(PUC) possesses an EcoRI site within the heterologous promoter region, and assuming there is no internal SstI site within the promoter fragment, one can digest the plasmid with both SstI and XbaI to remove the desired omega/heterologous promoter fragment. Those skilled in the art will realize that other cleavage sites are available for removing the omega/heterologous promoter from a pHKY(PUC) plasmid. That specific means for doing so are described here is not to be construed as limiting the invention in any way. Such other means are meant to be encompassed by the present invention. In any case, the desired excised omega/heterologous promoter fragment is inserted upstream from the promoterless polypeptide encoding region of the desired expression vector.

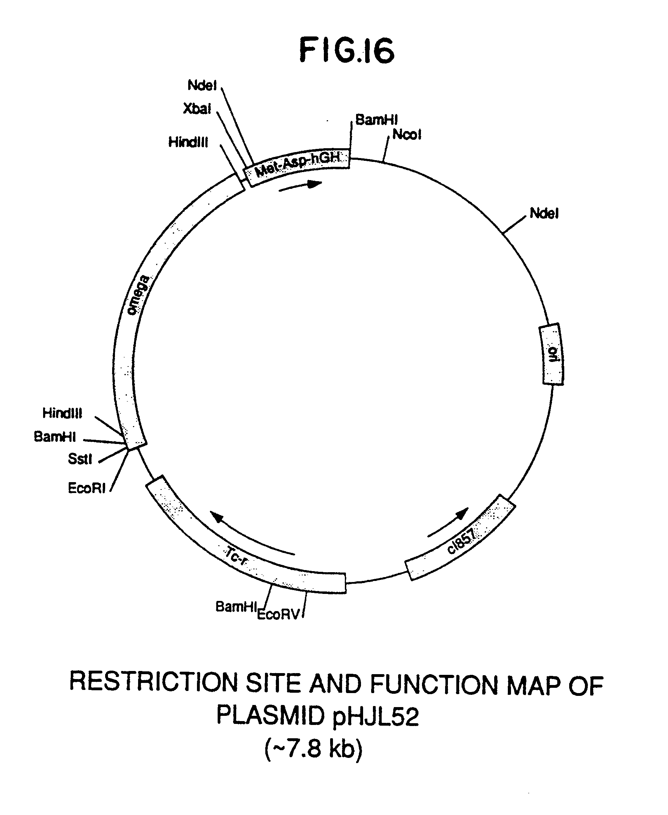

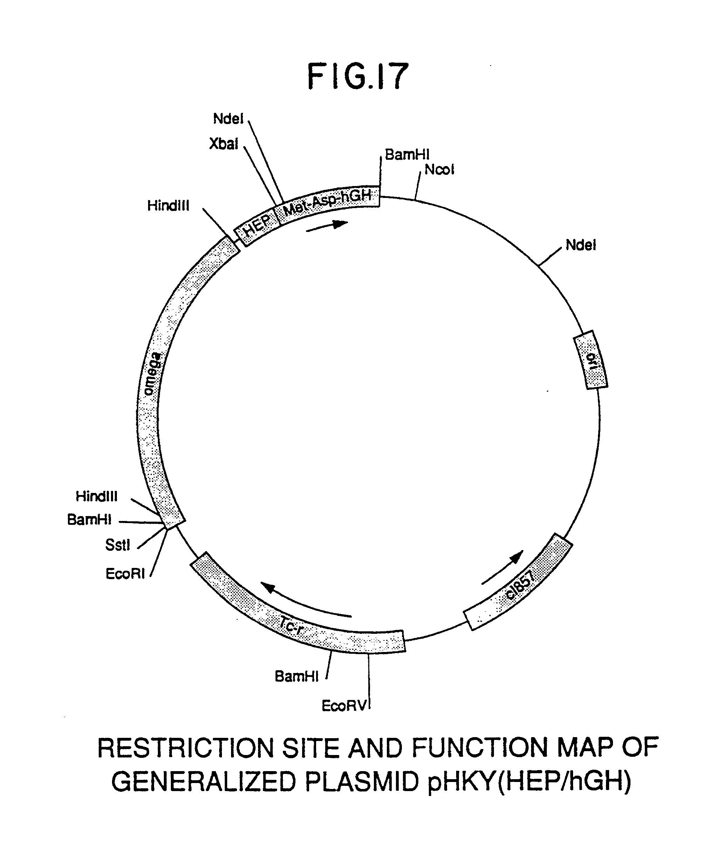

In one construction, plasmid pHJL52 can be digested with EcoRI and XbaI restriction enzymes if the heterologous promoter fragment to be inserted contains no internal EcoRI restriction site. Plasmid pHJL52 can be conventionally isolated from E. coli K12 C600/pHJL52 which has been deposited at the NRRL in accordance with the terms of the Budapest Treaty. A sample of this transformed host is available to one skilled in the art from the NRRL under the accession number NRRL B-18647 (deposited April 16, 1990). A restriction site and function map of plasmid pHJL52 is shown in Figure 16. Plasmid pHKY(PUC), likewise, is digested with EcoRI and XbaI and the desired omega/heterologous promoter fragment is purified and ligated with the fragment resulting from the similarly digested plasmid pHJL52. Alternatively, if the heterologous promoter fragment contains an internal EcoRI site, then plasmid pHKY(PUC) is digested with SstI and XbaI restriction enzymes to remove the omega/heterologous promoter fragment. This fragment then is ligated to the fragment resulting from SstI-XbaI digestion of plasmid pHJL52. The ultimate constructions, in either case, have been referred to as generalized plasmids pHKY(HEP/hGH). A restriction site and function map of generalized plasmid pHKY(HEP/hGH), which can be prepared in the manner described above, is shown in Figure 17.

In plasmid pHKY(HEP/hGH), the heterologous promoter is positioned upstream from the coding region for Met-Asp-hGH. Upon transformation of these plasmids into E. coli, the level of expression of the Met-Asp-hGH polypeptide can be determined.

Although Met-Asp-hGH was used to show the expression of a desired polypeptide under transcriptional control of the heterologous promoter of the invention, those skilled in the art will appreciate that any other DNA sequence encoding any polypeptide could be used for this purpose. For example, one skilled in the art will understand that the Met-Asp-hGH coding sequence could be substituted with genes encoding, for example, preproinsulin, proinsulin, insulin, individual insulin chains, for example, insulin A chain or insulin B chain, a growth hormone, such as bovine growth hormone or natural human growth hormone, a tissue plasminogen activator, a protein C, a protein S, an interferon, an immunoglobulin, a polypeptide hormone, a polypeptide enzyme, or a polypeptide antigen. Further, the polypeptide to be inserted could be selected from human sources or non-human sources such as a bovine or porcine source. In a preferred embodiment, the desired polypeptide is of human origin. Furthermore, the inserted gene encoding the polypeptide can be a gene encoding a fragment of a full length polypeptide. Additionally, the inserted gene can be synthesized in whole or in part using techniques which are now well known to those skilled in the art. Thus, totally synthetic or partially synthetic gene constructions are encompassed by the present invention. In addition, those skilled in the art are familiar with the degeneracy of the genetic code and thus one can prepare DNA sequences which produce the desired polypeptide even though the gene sequence is different from that obtained from natural sources. These modified sequences, like the natural sequences, can be totally or partially synthetic. For example, those skilled in the art are familiar with the means for "mutagenizing" a gene sequence, such as by site-directed mutagenesis, to delete or modify a given sequence to one which is desired. Thus, the present invention encompasses all of these alternative means for preparing a DNA sequence which encode a polypeptide.

Furthermore, in the preparation of these polypeptide encoding DNA sequences, or the promoters or promoter-containing DNA sequences of the invention, one may isolate various fragments for later ligation or recombination with other components of the vectors of the present invention. Indeed, as described above for the polypeptide encoding sequence, a promoter of the invention could be synthesized, in whole or in part, or modified as noted above. Thus, as used throughout this specification, "constructed" refers to any DNA sequence which is modified, synthesized (totally or in part), recombined or isolated in the preparation of a DNA fragment or vector useful in the present invention.

Likewise, the specific sequences outlined above in the especially preferred embodiment of the invention, although isolated from the genomic DNA of the natural source, can be synthesized, modified, recombined or isolated in the same manner as any "constructed" DNA sequence or vector. Further, the specific sequences noted earlier can be used to prepare, by means known by those skilled in the art, oligonucleotide hybridization probes which can be used to define other heterologous promoters of the invention. Promoters which are constructed in this fashion, likewise, are meant to be encompassed by the invention. Also, as used herein, "derivable" or "obtainable" is meant to connote that one source of the heterologous promoters is the genomic DNA from which they were removed. Once removed, however, the promoter containing sequence can be sequenced and synthesized in the manner described above. Thus, although the promoters may be "derivable" or "obtainable" from a given DNA source, other means for preparing that promoter such as those described above, will be apparent to those skilled in the art. Those other means for preparing the sequences are also encompassed by the term "derivable" or "obtainable" and any promoter containing sequence prepared by such means is meant to be encompassed within the present invention.

Table III provides a listing of especially preferred plasmids of the present invention and the specific plasmids prepared from each. The generalized plasmid names are provided at the tope of each column of the table and the specific plasmid names are listed below the general headings.

The following non-limiting examples are provided to further illustrate the invention. Although specific equipment, reagents, for example, restriction enzymes, etc. are disclosed in the examples, those skilled in the art will appreciate that alternate methods could be used without going beyond the intended scope of the invention. Thus, other restriction sites, reagents, or recombinant DNA linkers, for example, could be used in place of the specific enzymes disclosed.

## A. List of Solutions

The following solutions are referred to throughout the Examples and are presented here for clarity.

1.    P medium ($\sim$100 ml):

| | |
|---|---|
| Sucrose | 10.3 g |
| $K_2SO_4$ | 0.025 g |
| Trace element solution (see #3) | 0.2 ml |
| $MgCl_2 \cdot 6H_2O$ | 0.203 g |
| Water | 80 ml |

After autoclaving add:

| | |
|---|---|
| $KH_2PO_4$ (0.5%) | 1 ml |
| $CaCl_2 \cdot 2H_2O$ (3.68%) | 10 ml |
| (N-tris(hydroxymethyl)-methyl-2-aminoethane sulphonic acid), "TES" buffer, 0.25M, pH = 7.2 | 10 ml |

2.   L medium (~100 ml):

| | |
|---|---|
| Sucrose (10.3%) | 100 ml |
| TES buffer, pH 7.2 (0.25M) | 10 ml |
| $K_2SO_4$ (2.5%) | 1 ml |
| Trace element solution (see #3) | 0.2 ml |
| $KH_2PO_4$ (0.5%) | 1 ml |
| $MgCl_2$ (2.5M) | 0.1 ml |
| $CaCl_2$ (0.25M) | 1 ml |
| Lysozyme | 1 mg/ml |

The L medium is autoclaved after preparation

3.   Trace Element Solution (~1 l):

| | |
|---|---|
| $ZnCl_2$ | 40 mg |
| $FeCl_3 \cdot 6H_2O$ | 200 mg |
| $CuCl_2 \cdot 2H_2O$ | 10 mg |
| $MnCl_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| $H_2O$ | 1 l |

4.   R2 Regeneration Medium (~1 l):

| | |
|---|---|
| Sucrose | 103 g |
| $K_2SO_4$ | 0.25 g |
| Trace element solution | 2 ml |
| $MgCl_2 \cdot 6H_2O$ | 10.12 g |
| glucose | 10 g |
| L-asparagine $\cdot$ $1H_2O$ | 2.0 g |
| casamino acids | 0.1 g |
| Agar | 22 g |
| Water | to 700 ml |

After autoclaving add:

| | |
|---|---|
| $KH_2PO_4$ (0.05 g/100 ml) | 100 ml |
| $CaCl_2$ (2.22 g/100 ml) | 100 ml |
| TES Buffer (5.73 g/100 ml, pH = 7.2) | 100 ml |
| NaOH (5 N) | 1 ml |

5. T medium (~14.5 ml):

| | |
|---|---|
| Sucrose (10.3%) | 2.5 ml |
| Distilled water | 7.5 ml |
| Trace element solution | 20 µl |
| $K_2SO_4$ (2.5%) | 100 µl |
| $CaCl_2$ (5 M) | 217 µl |
| Tris-maleic acid, pH = 8 (1M) | 543 µl |
| Polyethylene glycol 1000 | 3.63 g |

All components were sterilized before use. The liquid components were mixed and then added to the appropriate amount of molten polyethylene glycol. The first four ingredients may be pre-mixed and stored at room temperature for at least one month.

6. Soft nutrient agar (SNA, ~1 l):

| | |
|---|---|
| Difco Bacto Nutrient Broth | 8 g |
| Agar | 5 g |

7. R2YE medium is R2 medium with 20 ml of 25% yeast extract added per liter.

8. Yeast Extract - Malt Extract (YEME, ~1 l):

| | |
|---|---|
| Yeast extract | 3 g |
| Peptone | 5 g |
| Malt extract | 3 g |
| Glucose | 10 g |

9. YEME + 34% Sucrose Liquid Complete Medium is YEME with 340 g/liter of sucrose.

10. YMX Media (~1 l):

| | |
|---|---|
| Yeast extract | 3 g |
| Malt extract | 3 g |
| Glucose | 2 g |
| Agar | 20 g |

11. Minimal Medium (MM):

| | |
|---|---|
| Agar | 10.0 g |
| L-Asparagine | 0.5 g |
| $K_2HPO_4$ | 0.5 g |
| $MgSO_4.7H_2O$ | 0.2 g |
| $FeSO_4.7H_2O$ | 0.01 g |
| Glucose (added after autoclaving) | 10.0 g |
| Distilled water | 1000 ml |

Place 2 g agar powder in each 250 ml Erlenmeyer flask; dissolve the other ingredients (except glucose) in the distilled water, adjust to pH 7.0-7.2 with NaOH, and pour 200 ml into each flask; close the flasks and autoclave. At time of use, re-melt the medium and add 4 ml of a 50% solution of glucose to each flask. Glucose can be replaced by alternative carbon sources such as glycerol (5 ml/l) or arabinose (5 g/l). ,L-asparagine is then replaced by $(NH_4)_2SO_4$ (2 g/l) as nitrogen source (to avoid confusion caused by the utilization of asparagine as carbon source).

12. R2 Soft-Agar Overlays:

| | |
|---|---|
| Sucrose | 103.0 g |
| $MgCl_2 \cdot 6H_2O$ | 10.12 g |
| $CaCl_2 \cdot 2H_2O$ (3.68%) | 100.0 ml |
| 0.25 M TES buffer, pH = 7.2 | 100.0 ml |
| Agar | 4.1 g |

Deionized water is added to bring the final volume to one liter.

Example 1

Isolation of Phage FP43 from Streptomyces griseofuscus C581 (FP43)

A. Phage Isolation

Phage lysates and DNA are prepared in substantial accordance with the procedure described in Cox and Baltz, 1984, J. Bacteriol. 159:499-504. A lyophilized culture of Streptomyces griseofuscus C581 (FP43) is obtained from the Northern Regional Research Center (NRRL), Agricultural Research Service, Peoria, IL 61604 under the accession number NRRL 18184 (deposited February 26, 1987). The lyophilized culture is used to inoculate 10 ml of NC broth. NC broth contains 8 g of Difco (P.O. Box 1058, Detroit, MI) nutrient broth per liter of deionized $H_2O$ and is supplemented with 4 mM $Ca(NO_3)_2$. The culture then is incubated at 29°C in a gyrotory incubator overnight (~16 hours). The culture is centrifuged to remove the cells and cellular debris; the supernatant then is passed through a 0.45 μ filter, and the filtrate containing phage FP43 particles is saved.

A lyophil of Streptomyces griseofuscus C581 is obtained from the American Type Culture Collection (ATCC), Rockville, MD 20852 under the accession number ATCC 23916. The lyophilized culture is used to inoculate 10 ml of TSB broth (Baltimore Biological Laboratories, Inc. (BBL), P. O. Box 243, Cockeysville, MD 21031) in a 50 ml flask. The culture is incubated at 29°C in a gyrotory incubator overnight.

Four 100 μl aliquots of the overnight culture of Streptomyces griseofuscus C581 are prepared and mixed, respectively, with 1.0 ml, 100 μl, 10 μl, and 1 μl of the lysate solution. The mixtures then are individually plated on NC agar (NC broth with 15 g/L agar) in 100 X 15 mm Petri plates and incubated at 34°C overnight. The following morning, the plates are examined, and the plate showing nearly confluent lysis is used to prepare the phage FP43 stock solution. The FP43 stock solution is prepared by adding ~5 ml of NC broth to the plate showing nearly confluent lysis, incubating the plate at room temperature for one to two hours, and collecting the broth from the plate. The solution is centrifuged and the resulting supernatant is passed through a 0.45 μ filter to remove debris. The resulting phage FP43 solution typically contains $10^9$ to $10^{10}$ FP43 particles per ml—the exact titer is determined by plating several dilutions of the phage stock on a sensitive strain, such as S. griseofuscus C581.

B. Phage DNA Isolation

To prepare phage FP43 DNA, the procedure described in Example 1A is followed, except the lysates are prepared using four to six 9.5″ X 9.5″ Petri dishes. The plates are washed with 50 ml of NC broth to collect the phage particles. The lysates are centrifuged and the supernatants passed through a 0.45 μ filter to remove cellular debris. The lysate then is centrifuged for 2 hours at 25°C at 30,000 rpm to pellet the phage particles. Each pellet is resuspended in 1 ml of φ buffer. φ buffer is 10 mM TES ("2-[(tris)-[hydroxymethyl]-methyl)amino]ethanesulfonic acid") and 10 mM $Ca(NO_3)_2$. This solution is centrifuged in a tabletop centrifuge to pellet material that did not go back into solution. The supernatant, in a manner analogous to the purification of bacteriophage lambda (see Maniatis et al., "Molecular Cloning: A Laboratory Manual", Cold Spring Harbor Laboratory (1982), pp. 80-81), is then layered on top of a CsCl gradient composed as follows (from most dense to least dense): 1.7, 1.6, and 1.4 g/ml in buffer. The gradient is prepared in a polyallomer tube ($\frac{1}{2}$″ X 2″), which was placed in an SW50.1 rotor (Beckman Instruments, Inc., Spinco Division, P. O. Box 10200, Palo Alto, CA 94304). The solution is centrifuged at 25,000 rpm at 15°C for 1 hour and yields two bands: a brown-tinged top band and a blue-tinged lower band. The lower band is collected with a syringe and dialyzed against 2 to 3 liters of φ buffer overnight.

The dialyzed band is extracted for 30 minutes with 1.5 volumes of φ buffer-saturated phenol (25 ml phenol:10 ml φ buffer) and then re-extracted for 10 minutes with another 1.5 volumes of φ buffer-saturated phenol. The phage band then is extracted 3 times with one volume of ether. The phage DNA is precipitated with 0.1 volume of 3 M sodium acetate (NaOAc), pH 8.0, and 2 volumes of absolute ethanol. The phage FP43 DNA is precipitated by centrifugation, washed with 70% ethanol and resuspended in ~ 1 ml of TE buffer (10 mM Tris-HCl, pH 8, 1 mM $Na_2$ EDTA), yielding a solution containing ~0.5 mg/ml of phage FP43 DNA.

EXAMPLE 2

Preparation of Phage FP22 and FP22r4 DNA

A. Phage Isolation

A frozen stock of actinophage FP22 or FP22r4, have been deposited under the terms of the Budapest Treaty and may be obtained from the ATCC ("American Type Culture Collection, 12301 Parklawn Drive, Rockville, MD 20852) under the accession numbers ATCC 40798 and ATCC 40799 (both deposited April 25, 1990), respectively. These stock should contain phage (frozen in Nutrient Broth ("NB") + 7% DMSO) at titers of approximately $10^9$ to $10^{10}$ pfu/ml. S. griseofuscus is grown according to Example 1A as an overnight culture in TSB broth. The phage stock is titered by thawing and making serial dilutions in NB. Aliquots of these dilutions are mixed with S. griseofuscus C581 in accordance with Example 1A and the precise titer of the phage determined.

B. Preparation of DNA

An overnight culture of S. griseofuscus C581 is grown as previously described. Suitable dilutions of the phage are made so that approximately 20,000 phage are mixed with 1 ml of the S. griseofuscus C581. To this mixture, 5 ml of NC soft agar overlays are added and plated on NC agar in a Petri dish (150 X 15 mm). This procedure is used to prepare 10 plates of phage. The plates are incubated overnight at 37°C. NC broth (50 ml) is added to each plate and these are kept at room temperature for 1-2 hours. Each plate is massaged gently with a microscope slide and the broth collected into suitable Sorvall centrifugation bottles. These are centrifuged at 10,000 r.p.m. to remove the cellular debris. The supernatants are filtered through a 0.45 μ filter and pooled together.

To the phage suspension, 1 mg of DNAse I is added. The reaction is placed at 37°C for 30-60 minutes. Sodium chloride is added to the phage suspension (2.9 g/100 ml) and the suspension stirred gently until the salt is dissolved. Polyethylene glycol 8000 (Sigma) (10 g/100 ml) then is added and gently stirred into solution. The mixture is transferred to Sorvall bottles and these are placed on ice for 1-2 hours. The PEG/phage precipitate is collected by centrifugation at 10,000 r.p.m. for 15 minutes. The pellet is resuspended in 15 ml of phage buffer. This is clarified by centrifugation. Solid cesium chloride is added to the phage suspension (0.819 g/ml) and the mixture gently agitated until all the cesium is in suspension. The density of the mixture is adjusted to a refractive index of 1.38. This phage/CsCl suspension is transferred to two 5/8" X 3" quick-seal polyallomer ultracentrifuge tubes (Beckmann), overlayed with mineral oil to fill the tubes, sealed and centrifuged at 50,000 r.p.m. in a Ti70 Beckman rotor for at least 24 hours until equilibrium is reached.

The tubes are removed and the bluish-white phage bands are removed with a syringe from each tube. These are pooled and dialyzed against 2-3 liters of phage buffer. The phage DNA is obtained by extraction two times with an equal volume of buffer saturated phenol followed by ether extractions (twice). The DNA is in the aqueous phase and is concentrated by precipitation. A 0.1 volume of 3M NaOAc, pH 8, is added to the DNA followed by 2 volumes of ethanol. This mixture is stored at -20°C overnight. The DNA precipitate is collected by centrifugation, the pellet washed once with 70% ethanol, and the DNA redissolved in approximately 1 ml of TE to a concentration of ~1 mg/ml.

EXAMPLE 3

Preparation of Streptomyces griseofuscus DNA

A 10 ml culture of S. griseofuscus C581 is grown in TSB overnight in substantial accordance with Example 1A. This culture is homogenized and used to inoculate 200 ml of the same media which has been supplemented with glycine to 0.4%. This culture is grown overnight. The mycelial fragments are harvested by centrifugation (Sorvall GSA in bottles) at 10,000 r.p.m. for 10 minutes. The cell pellet is resuspended in 100 ml of TE + 25% sucrose containing lysozyme at 0.5 mg/ml. This reaction is incubated at 37°C for 1 hour. Fifty ml of 0.25M EDTA (pH 8) is added and the mixture divided into 25 ml aliquots into Sorvall tubes. To each tube 1 ml of a 20% SDS (Fisher) solution is added and the tubes gently agitated at room temperature until lysis occurs (about 1 hour). 5M NaCl (8 ml) is mixed into the solution. The tubes are placed on ice for about 2 hours. The tubes are centrifuged at 15,000 r.p.m. for 15 minutes and the supernatants pooled. -Isopropanol (0.64 volumes) is mixed into the solution and the precipitate collected by centrifugation and air dried. This precipitate is resuspended in 5 ml of TE by gentle agitation and heating to 55°C. This mixture is centrifuged in a benchtop centrifuge to remove the cellular debris. To this mixture, 100 μl of a RNase A (2 mg/ml in 50 mM Tris (pH 8); Boehringer Mannheim, Indianapolis, IN, prepared as a stock solution as noted in Example 4B) is added and the reaction incubated at 37°C for about 1 hour. The mixture is gently extracted 2 times with an equal volume of phenol and then extracted two times with ether. Sodium acetate (3M, 0.2 volume) is then added followed by 2 volumes of ethanol. This is placed at -20°C overnight. The DNA precipitate is collected by centrifugation, washed with 70% ethanol, air dried, and redissolved in 1 ml of TE to a final concentration of ~1 mg/ml.

EXAMPLE 4

A. Preparation of Plasmid pHJL35

Two μl of Plasmid pHPR66 (.25 μg/μl) in 2 μl of 10X HindIII buffer (0.5M NaCl; 0.5M Tris-HCl (pH 8.0); .1M MgCl₂;) were added to 2 μl bovine serum albumin ("BSA") (1 mg/ml), 13 μl of distilled H₂O and 1 μl (~10 units) of restriction enzyme HindIII. The cleavage reaction is allowed to proceed for about 2 hours at about 37°C.

Four μl of the omega fragment (.1 μg/ml) (obtained as a ~2.1 kb SmaI fragment from Amersham Corp., Arlington Heights, IL) were added to 2 μl of 10X HindIII buffer (as defined above), 2 μl BSA,(1 mg/ml), 1 μl (~10 units) of restriction enzyme HindIII and about 11 μl of distilled H₂O. The reaction mixture was incubated for about 2 hours at about 37°C.

After the reactions had gone to completion, the digestion was stopped by incubating the reaction mixtures at about 70°C. After this, the DNA was co-precipitated by the addition of about 10 μl of the plasmid pHPR66 digest to the the omega fragment digest mixture. To this mixture was added 25 μl of distilled H₂O, 6 μl of 3M sodium acetate (pH 8), and 120 μl of 100% distilled ethanol. The mixture was mixed thoroughly, placed on dry ice for 15 minutes and the precipitate collected by centrifugation for 8 minutes. The supernatant was discarded, the tube swabbed to remove the liquid, and the tube dried. The DNA was redissolved in 6 μl H₂O, 3 μl of 5X ligase/kinase buffer (250 mM Tris-HCl (pH 7.8), 50 mM MgCl₂, 25 mM dithiothreitol ("DTT"), 25% glycerol), 5 μl ATP (.66 mM (pH 8.0)), and 1 μl T4 DNA ligase (~1 units/μl). This reaction mixture was incubated overnight at 15°C. A sample (5 μl) of the ligation product was used to transform E. coli K12 MM294 in the general manner described below in Example 5, for E. coli K12 C600, except that E. coli MM294 is used. The transformants were selected using ampicillin (50 μg/ml), and replica-plated to plates containing streptomycin (50 μg/ml) and chloramphenicol (5, 10, 25, 50, 75 and 100 μg/ml) and ampicillin. Colonies which were chloramphenicol sensitive and ampicillin/streptomycin resistant were examined by "in the well lysis", as described below. Those clones having the desired phenotype and size, as determined by the in the well lysis, were "miniprepped" (see protocol below). Restriction analysis using AccI, BamHI, HindIII, and HincII, allowed identification of plasmid pHJL35, the restriction site and function map of which is shown in Figure 1. Plasmid pHJL35 can be isolated by conventional means from E. coli K12 MM294/pHJL35 which is a strain deposited in the Northern Regional Research Center ("NRRL"), Peoria, Illinois 61604. This strain is available from the NRRL under the accession number NRRL B-18645.

B. In the Well Lysis

A 1% horizontal agarose gel is prepared. To 1 ml. of lysis solution #1 (.05% Bromophenol blue; 20% sucrose in 1 X TBE buffer [5X TBE buffer is 80 g. Sigma 7-9™ (Sigma Chemical Co., St. Louis, Missouri 63178); 27.5 g boric acid; 4.62 g sodium EDTA; add water to prepare 1 liter], is added 50 μl of lysozyme mix and 25-50 μl of RNase A solution. RNase A solution is prepared as a 2 mg/ml stock solution in 50 mM Tris (pH 8.0), boiled 10 minutes and frozen in 50 μl aliquots at -20°C. Lysozyme is prepared as a 5 mg/ml stock solution in 50 mM Tris (pH 8.0) and stored at -20°C in 50 μl aliquots. To each well is added 15 μl of lysis solution #1. The desired colony is picked with a toothpick, placed in the well and swirled into suspension in the solution. For standards, cleaved lambda DNA and/or uncut vector DNA is used. Next, 15 μl of lysis solution #2 (0.2% SDS, 10% sucrose in 1 X TBE buffer) is added to each well and the solution is stirred twice with a fresh toothpick. After at least about 5 minutes, each well is overlaid with lysis solution #3 (0.2% SDS, 5% sucrose in 1 X TBE buffer). The DNA is electophoresed at 5-6 milliamps for 30 minutes and then at 40-50 milliamps (150 volts) for two to three hours making certain that the wells do not dry out by refilling them occasionally with lysis solution #3. The gels are visualized by staining with a dilute solution (2 μg/ml) of ethidium bromide and exposing the stained gel to U.V. light. The size of the plasmids in each colony is compared to the standards and those of the desired size are isolated and examined further.

C. Miniprep DNA Protocol

A 10 ml shake flask culture is grown overnight at about 30°C to about 37°C in L broth containing the appropriate antibiotic. This culture then is centrifuged and the pellet is resuspended in 50 mM Tris-HCl (500 µl) containing lysozyme (1 mg/ml). This mixture is incubated at room temperature for 20 minutes after which 500 µl of buffer saturated phenol is added, mixed three times and centrifuged for 15 minutes. The aqueous layer is removed to a new tube, 300 µl of buffer saturated phenol is added. The mixture is vortexed and then centrifuged for 5 minutes after which the phenol is carefully removed. The DNA solution is extracted three times with ether followed by the addition of 1 ml of cold absolute ethanol. The mixture then is mixed and placed for 5 minutes on dry ice. The precipitate is collected by centrifugation, dried in vacuo and redissolved in 100 µl of distilled $H_2O$. This DNA then can be digested in a standard restriction digest by including the RNase A solution described in part B, above.

### D. General Procedure for Gel Purification of DNA

The particular enzymatic reaction is performed as desired. A 1% TBE agarose gel is used to prepare a preparative well. The DNA to be purified is applied to the gel using a standard running dye additive. Care is taken to distribute evenly the digested DNA over the entire preparative well. Depending on the size of the DNA fragment, the amount of DNA loaded and length of the run may vary. For example, a DNA fragment very close in size to the undesired fragment may require the loading of a full length well and running overnight. This generally allows DNA which is as close as about 500 bp to be separated if the size ranges from about 1 to about 6 kb. Larger fragments may require the use of less DNA and perhaps a lower concentration of gel. Small fragments or those which are fairly unique may be isolated on quick gels. Quick gels are generally run from about 1 to about 3 hours at 150 volts while overnight gels are run for about 14 to about 18 hours at 25-50 volts following an initial 15 minute run at 150 volts.

The gel then is stained with ethidium bromide (1.5 µg/ml) for 30 minutes in distilled water. The gel is then visualized with U.V. light and small slits are made directly in front of and behind the desired fragment. Small pieces of DEAE-cellulose paper (Whatman) are cut to fit in these slits so that they protrude just above the surface of the gel. The paper then is placed carefully in the gel. The gels are then placed in the buffer tank so that the buffer just reaches the top of the gel. The gel then is electrophoresed at 150 volts for about 15 minutes after which the DNA should have moved into the paper.

The paper is placed in a 15 ml centrifuge tube and washed gently with 5 ml of wash buffer (10 mM Tris (pH 8.0), 100 mM KCl). The wash is discarded. Five ml of elution buffer (10 mM Tris (pH 8.0), 1 M NaCl) is added and the paper is macerated by shaking or vortexing. The elution mix is filtered through a syringe containing siliconized glass wool and a Schleicher-Schuell (Keene, N.H. 03431) Elutip Pre-filter into a 15 ml centrifuge tube. Absolute ethanol (10 ml) is added, the mixture is mixed and placed at -20°C overnight. The DNA then is collected by centrifugation in a HB4 Sorvall rotor, dried, and then redissolved in 100 µl of distilled water. The amount of DNA can be quantitated by visualization on a small agarose gel or by use of a spectrophotometer using standard methods.

### Example 5

### A. Selection of Heterologous Promoters Using Promoter Probe Vector Plasmid pHJL35

### I. Construction of Generalized Plasmids pHKY(HEPS)

Genomic DNA from Streptomyces griseofuscus C581, as prepared in Example 3, actinophage FP43 (prepared as in Example 1), FP22 or FP22r4, as prepared in Example 2, was used to prepare heterologous promoter containing plasmids using plasmid pHJL35 as the promoter probe. Twenty micrograms of the desired DNA are cleaved to completion with TaqI restriction enzyme (New England BioLabs, Beverly, MA 01915) according to the manufacturer's recommendations. The digestion reactions are extracted twice with phenol saturated with buffer (50 mM Tris-HCl (pH 8.0)) and twice with ether.

Plasmid pHJL35 (43 µg), prepared as in Example 4, is cleaved with AccI restriction enzyme (New England BioLabs, Beverly, MA 01915) according to the manufacturer's recommendations except that the digestion is carried out at 55°C. The reaction volume was raised 2X with 50 mM tris (pH 8.0) and 4 units of calf intestinal alkaline phosphatase ("CIAP" - Boehringer Mannheim, Indianapolis, IN 46250) were added. This reaction is incubated at 37°C for 30 minutes and then for 15 minutes at 70°C. The mixture then is extracted with phenol and ether as described above.

The DNA from each of the digestions is precipitated, dried and then resuspended in TE buffer (10 mM Tris-HCl (pH 8.0), and 1 mM EDTA) to prepare a final concentration of about 0.1 µg/µl. In the case of Streptomyces griseofuscus and actinophage FP22 or FP22r4, 14 µl of the digested DNA is co-precipitated with 33 µl of Ac-

EP 0 456 507 A2

cI-digested plasmid pHJL35. In the case of actinophage FP43, 9 μl of the digested FP43 DNA is co-precipitated with 35 μl of AccI-digested plasmid pHJL35.

After drying the precipitates, each of the Streptomyces griseofuscus/pHJL35, FP22r4/pHJL35, or FP-22/pHJL35 mixtures is redissolved in 20 μl of the following ligation reaction mixture: 14 μl 5X ligase/kinase buffer (250 mM Tris-HCl (pH 7.8); 50 mM MgCl$_2$; and 25 mM DTT, 10 mM ATP (45 μl); 1 μl of T4 DNA ligase (1 unit/μl-Boehringer Mannheim, Indianapolis, IN 46250) and 5 μl of distilled H$_2$O. In the case of the FP43/pHJL35 digestion mixture, the DNA is redissolved in 10 μl of the ligation reaction mixture. The reactions were incubated at 15°C for at least 18 hours after which the reaction was stopped by raising the temperature to 70°C for 10 minutes. One μl of the ligation mixture was diluted with 99 μl of 0.1X SSC (0.015 M NaCl, 0.0015 M sodium citrate at pH 7.0). The resulting ligation products, referred to as generalized plasmids pHKY(HEPS), are transformed into E. coli in the manner described below.

## II. Transformation into E. coli

### A. Preparation of Frozen Competent E. coli K12 C600R$_k$-M$_k$-

Fresh overnight cultures of E. coli K12 C600R$_k$-M$_k$-, a strain widely available and on deposit with the ATCC under the accession number ATCC 33525, are subcultured 1:10 in fresh L-broth (disclosed in Miller, 1972, Experiments in Molecular Genetics, Cold Spring Harbor Labs, Cold Spring Harbor, N.Y.) and grown at 37°C for 1 hour. A total of 660 Klett Units of cells are harvested, washed with 2.5 ml of 100 mM NaCl$_2$, suspended in 2.5 ml of 150 mM CaCl$_2$ and incubated at room temperature for 20 minutes. The cells are harvested by centrifugation, resuspended in 0.5 ml of 150 mM CaCl$_2$-10% glycerol, chilled on ice for 3-5 minutes and frozen. The suspensions of cells are stored in liquid nitrogen until use. Preservation and storage do not adversely affect the viability or frequency of transformation by covalently closed circular DNA.

### B. Transformation

The competent cells are thawed in an ice bath and mixed in a ratio of 200 μl of cells to 1 μl of heat killed ligation reaction mixture, prepared as above, and to which 99 μl of 0.1X SSC have been added. The transformation mixture was chilled on ice for 20 minutes, heat shocked at 42°C for one minute, placed on ice for 10 minutes, and L broth added to bring the volume to 2 ml. The transformed cells then are incubated for 90 minutes at 37°C and then plated at .2 ml/plate on L-agar containing ampicillin (50 μg/ml). The plates are incubated at 37°C for 18 hours. The cells then are replica plated, using sterile velvets, to L-agar containing streptomycin (50 μg/ml); L-agar containing chloramphenicol at 100, 250, 500, 1000, and 1500 μg/ml; and finally L-agar containing ampicillin at 50 μg/ml. Those colonies which are resistant under these selection criteria contain a heterologous promoter of the invention and are selected for further analysis.

## Example 6

## Transformation of Heterologous Promoter Containing Clones into Streptomyces

### A. Preparation of Generalized Plasmids pHKY(TN5)

To prepare generalized plasmids pHKY(TN5), the TN5 aphII gene is removed from plasmid pIJ486 (Bibb et al., Mol. Gen. Genet., 203 468 (1986)) derived from a S. lividans TK23 strain using a Standard SDS lysis protocol (see, for example, Hopwood, D.A., et al., "Genetic Manipulation of Streptomyces: A Laboratory Manual", The John Innes Foundation, Norwich, England (1985)). The aphII gene is isolated via paper electrolution techniques on a BclI restriction fragment, using standard methods as described above. Plasmid pUC19, cleaved with restriction enzyme BamHI, and the aphII-gene containing BclI fragment are ligated to prepare plasmid pHJL45. Plasmid pHJL45 has been transformed into E. coli K12 JM109, from which it can be isolated by conventional means and has been deposited (April 16, 1990) at the NRRL from which it is available under the accession number NRRL B-18646.

To prepare the plasmids generally designated pHKY(TN5), the desired plasmid pHKY(HEPS), prepared in accordance with Example 5, is cleaved by standard methods with restriction enzymes XbaI and SstI. The heterologous promoter containing fragment is isolated by standard gel purification techniques, as described earlier. Likewise, plasmid pHJL45 is treated with XbaI and SstI and the fragment containing the aphII gene is isolated by gel purification.

The XbaI/SitI heterologous promoter containing fragment and the XbaI/SstI aphII containing fragment are ligated under standard conditions. E. coli K12 C600 transformants prepared in substantial accordance with the teaching of Example 5, were screened for resistance to ampicillin, streptomycin and kanamycin. Those clones expressing the proper phenotype are analyzed to ensure that the heterologous promoter and aphII gene are present. Those vectors with the proper construction are designated with the generalized plasmid name, plasmid pHKY(TN5). A restriction site and function map of plasmid pHKY(TN5) is provided in Figure 4.

B. Preparation of Generalized Plasmids pHJL(HEP/TN/302), pHJL(HEP/TN/400, and pHJL(HEP/TN/401)

Starting plasmids pHJL302, pHJL400 and pHJL401 are isolated, by conventional means (see, for example, Maniatis, T., et al., "Molecular Cloning: A Laboratory Manual", Cold Spring Harbor Laboratory, Cold Spring Harbor, N.Y. (1982)) from E. coli K12 JM109/pHJL302 NRRL B-18648, E. coli K12 JM109/pHJL400 NRRL B-18650, or E. coli K12 JM109/pHJL401 NRRL B-18217, respectively. These shuttle vectors contain the SCP2* replicon which allow replication in Streptomyces host strains (see, U.S. Patent No. 4,752,574, incorporated herein by reference).

The desired starting vector is conventionally cleaved with BamHI and SstI restriction enzymes and the SCP2* replicon containing fragment is gel purified.

Likewise, to remove the omega fragment/heterologous promoter/TN5 fragment, the desired plasmid pHKY(TN5), prepared in Part A, is cleaved by conventional means with BamHI and SstI restriction enzymes and the desired fragment is gel purified and ligated to the BamHI/SstI cleaved plasmid pHJL302, pHJL400, or pHJL401. The ligated product is transformed into E. coli K12 JM109 in the following manner:

C. General Protocol for Transformation into E. coli K12 JM109

The following Procedures A and B are alternate descriptions of how to prepare E. coli K12 JM109 cells that are competent for transformation. In particular, lyophils of E. coli K12 JM109 are obtained from the ATCC under the accession number ATCC 53323.

I. Procedure A

E. coli K12 JM109 ("JM109") is streaked on M9 glucose plates. M9 minimal medium is prepared by autoclaving 18 g Bacto agar in 850 ml dH$_2$O, cooling to 50°C and then adding the following solutions:

| | |
|---|---|
| 100 ml | 10X M9 salts, |
| 10 ml | .1M MgSO$_4$, |
| 10 ml | .01M CaCl$_2$, |
| 5 ml | 1.0 mg/ml B1, |
| 10 ml | 40% glucose |

10X M9 salts are prepared in distilled water with 60 g Na$_2$HPO$_4$, 30 g KH$_2$PO$_4$, 5 g NaCl, and 10 g NH$_4$Cl. The volume is brought to 1.0 liter, the pH is adjusted to 7.4, and the solution then is autoclaved. A single colony is picked from the M9 plate and is used to inoculate 10 ml M9 glucose (same media as above without the agar). The culture is grown overnight at 30-37°C. One ml of the culture is inoculated into 200 ml L broth in a 1 liter flask and grown at 37°C with good aeration. Growth is followed at OD$_{550}$. Because turbidity measurements generally are not accurate above .4 OD samples should be diluted to be within a .1-.4 OD range. When the culture reaches OD$_{550}$ .9-1.0, the flask is placed in an ice-water bath with occasional swirling for 10 minutes. The temperature for the remaining steps is maintained at 0°C. The cells are centrifuged in a GSA rotor for 10 minutes at 5000 r.p.m. The cell pellet is resuspended in 100 ml Solution A (.01M MOPS buffer (pH 7.0), .01M RbCl) and centrifuged again for 10 minutes at 5000 r.p.m. The cell pellet then is resuspended in 100 ml Solution B (.1M MOPS buffer (pH 6.5), .05M CaCl$_2$ and .01M RbCl) and incubated on ice for 15 minutes, followed by centrifugation at 5000 r.p.m. for 10 minutes. The cell pellet should be diffuse. The pellet is resuspended in 15 ml of Solution B, and incubated on ice in the refrigerator overnight. To this suspension is added 5 ml 50% glycerol. The mixture then is mixed and frozen at -70°C in .05 ml aliquots.

II. Procedure B

An overnight culture of E. coli K12 JM109 (10 ml), prepared as in Procedure A, is added to 1 liter of L broth. The culture is split into four one liter flasks and grown at 37°C to an OD$_{500}$ of 0.5. The cultures are then removed to Sorvall bottles and put on ice for 10 minutes, followed by centrifugation at 3000 r.p.m. for 5 minutes. Fifty ml of Solution C (60mM CaCl$_2$, 15% glycerol) is added to each bottle and the mixtures are combined, centrifuged at 3000 r.p.m. for 5 minutes, and the cell pellet is resuspended in 200 ml of Solution C, then set in ice for 30 minutes. The mixture is centrifuged at 3000 r.p.m. for 5 minutes, the pellet is resuspended in 40 ml of Solution C, and the mixture is stored on ice at 4°C overnight. Aliquots are prepared at 0.5 ml/vial and stored at -70°C.

### III. Transformation

The frozen competent cells, prepared by either Procedure A or B, above, are thawed. Five μl of the desired ligation reaction are mixed with 95 μl of competent cells. The cell-DNA mixture is incubated on ice for 20 minutes and heat shocked at 42°C for 1 minute. The mixture is incubated on ice for about 10 minutes. The mixture is diluted to 1 ml with L broth and incubated at 30°C for 90 minutes. The mixture then is plated on suitable agar plates and incubated at 30°C overnight.

As noted earlier, insertion of DNA into a restriction site of the polylinker on the parent plasmid, disrupts the lacZ α-fragment coding sequence and concomitantly destroys the ability of the pHJL302, pHJL400, or pHJL401 derivative to complement a ΔM15-type mutation. β-Galactosidase can hydrolyze X-Gal which is 5-bromo-4-chloro-3-indolyl-β-D-galactopyranoside, a colorless compound, to an indigo-colored product and thus allows for a convenient screening method for discriminating between transformants containing starting plasmid or modified plasmid, such as plasmids pHJL(HEP/TN/302), pHJL(HEP/TN/400), or pHJL(HEP/TN/401).

Thus, aliquots of the transformation mixture, prepared according to the methods described above, are plated on L-agar plates containing 100 μg ampicillin/ml and 40 μg X-gal/ml (50 mg/l N,N-dimethyl-formamide). The plates are incubated at 37°C overnight. Colonies that contain a plasmid with an insert are white, whereas parental transformants are indigo-colored on these plates.

Transformants which are white on X-gal medium and which are resistant to ampicillin, kanamycin and streptomycin are selected and subjected to the mini-prep protocol described in Example 4. These resulting E. coli/Streptomyces shuttle vectors, designated pHJL(HEP/TN/302), pHJL(HEP/TN/400) or pHJL(HEP/TN/401), depending on the parent plasmid and which contain the desired construction are transformed, as described below, into Streptomyces lividans TK23 (available from the NRRL under the accession number B-15826, deposited July 27, 1984) and the level of resistance to kanamycin is determined.

### D. Transformation into Streptomyces lividans

The vector prepared in Section B, above, can be transformed into Streptomyces lividans TK23 using the following protocol:

### I. Preparation and Storage of Protoplasts

Streptomyces lividans TK23 (NRRL B-15826) is grown for 40-48 hours at 30°C in YEME + 34% sucrose, 5 mM $MgCl_2$ and 0.5% glycine. YEME + 34% sucrose is YEME media with 340 g/liter of sucrose. YEM (~1 l) contains 3 g yeast extract, 5 g Peptone, 3 g malt extract, and 10 g glucose, with water added to make 1 liter. The mycelia are recovered by centrifugation (800Xg for 10 minutes in a bench top centrifuge) and washed twice in 10.3% sucrose. The mycelia from 25-50 ml of culture are suspended in 3-4 ml of L medium and incubated for 1 hour at 32°C. During this interval the suspension is pipetted up and down once or twice to disperse clumps. Five ml of P medium are added, and the suspension is filtered through a plug of cotton wool. The protoplasts are then suspended in 4 ml of P medium and the number of protoplasts may be determined microscopically using a hemacytometer slide. If the protoplasts are not to be used immediately, the suspension can be divided into aliquots (about 1 ml) containing $5 \times 10^9$-$10^{10}$ protoplasts in sterile polypropylene screw-cap tubes. The suspensions are frozen slowly by placing the tubes in a container of ice, which was in turn placed at -70°C. The protoplasts are stored at this temperature until needed. The frozen suspension then is thawed rapidly by immersion in a 37°C water bath prior to use.

### II. Protoplast Transformation

Approximately $5 \times 10^9$ protoplasts are pelleted by centrifugation (800Xg for 10 minutes). The supernatant is decanted and the protoplasts are resuspended in the small volume of liquid remaining in the tube. Plasmid pHJL(HEP/TN/302), pHJL(HEP/TN/400), or pHJL(HEP/TN/401), in a volume not greater than 20 μl in TE buffer, is added followed immediately by the addition of 0.5 ml of T medium. The mixture is pipetted up and down once or twice to mix the contents. At this point the suspension is either plated directly or diluted with 0.5 ml of P medium and then plated. In either case, about 0.1 ml is inoculated per plate of R2YE medium. These are grown overnight at room temperature. The plates are overlaid with 3 ml R2 overlay media containing 30 μl thiostrepton (40 mg/ml in DMSO) and incubated at 30°C for 2-3 days.

Example 7

Comparison of Polypeptide Expression by Vectors Containing Heterologous Promoters

The ability of the heterologous promoters to express high levels of a polypeptide was tested by using generalized vectors pHKY(HEP/hGH). These vectors contain a heterologous promoter positioned to drive transcription of an inserted Met-Asp-hGH encoding sequence. The method for preparing these vectors has been described earlier. The ability of plasmids pHKY(HEP/hGH) to express the Met-Asp-hGH product was compared to an isogenic vector, plasmid pCZR340, which contained the bacteriophage lambda pL ($\lambda_{pL}$) promoter in place of the heterologous promoter. Prior to the present invention, it was believed that $\lambda_{pL}$ was the strongest promoter known to occur naturally in E. coli. After transformation in a modifying strain, as described below for the shake flask fermentations, each of the noted vectors were transformed into E. coli K12 RV308, using the protocol described in Example 5 for E. coli K12 C600, except that E. coli K12 RV308 is substituted. The transformed strains then are fermented in 10 L fermenters in complex media. E. coli K12 RV308 is deposited (September 28, 1983) under the terms of the Budapest Treaty and is available from the Northern Regional Research Laboratory under the accession number NRRL B-15624. In particular, these experiments were completed in L broth prepared according to the following formula:

100 g      tryptone
50 g      Bacto-yeast extract
50 g      NaCl
5 ml      Sag 5693 (antifoam)
0.05 g      tetracycline
10 L      deionized water

The medium was sterilized at 121°C for 30 minutes, then cooled to 30°C for inoculation. One ml of a frozen vegetative inoculum, prepared from a recloned colony, was used to inoculate a 500 ml L broth vegetative growth flask containing 5 μg/ml tetracycline. The inoculated broth was incubated at 30°C for approximately 9 hours. One hundred ml of this culture was used to inoculate the sterile fermentation medium. The fermentation conditions at inoculation were the following: temperature, 30°C, back pressure = 5 psig, dissolved oxygen >95%. No attempt to control pH was made. No induction conditions were specifically implemented. The fermentation can be carried out at 30°C to 37°C. Accumulation of cell mass and product formation was allowed to continue for approximately 14-16 hours depending upon the experiment. Table IV shows the levels of expression for several of the heterologous promoter containing vectors of the invention when fermented under these conditions.

Similar studies were performed using Shake Flask Fermentations. To do so each of the noted vectors were transformed into E. coli K12 MM294 using the general protocols of Example 5. These were selected on L agar containing tetracycline (12.5 μg/ml). Colonies of the desired phenotype were examined by the miniprepping technique described earlier and restriction mapping performed to determine proper structure of the plasmid DNA in the minipreps. The plasmid minipreps then were transformed into E. coli RV308 according to established protocols. Transformation into E. coli K12 MM294 prior to RV308 is necessary because starting plasmid pHJL52 DNA was prepared from E. coli K12 C600 which is r⁻m⁻ and would be restricted in E. coli K12 RV308 (which is r⁻m⁺). Therefore, DNA must be modified in E. coli K12 MM294 (a r⁻m⁺ genotype) before transformation into E. coli K12 RV308. Transformants were selected for resistance to tetracycline and then tested for resistance to streptomycin and spectinomycin. Colonies of the proper phenotype (i.e. resistance to all three antibiotics) were miniprepped and restricted to confirm proper plasmid structure. These were then single colony streaked out on L agar containing tetracycline to use as source for fermentations.

Shake flask fermentations were carried out using the following protocols: A single colony was used to inoculate a 50 ml flask containing L broth and tetracycline and grown overnight at 30°C. This culture (15 ml) was used to inoculate 135 ml of the same media. All Met-Asp-hGH/heterologous promoter plasmid containing cultures were grown at 39°C for 6 hours. The control strain contains a thermo-inducible lambda pL promoter and thus is grown initially at 30°C for two hours then shifted to 39°C for 4 hours. A sample (14 ml) of each culture was removed and the dry weight was determined. A sample (100 ml) was removed and centrifuged at 10,000 r.p.m. for ten minutes. The supernatants were discarded, the cell pellets washed in 50 ml of 50mM Tris (pH 8), and the centrifugation repeated. The cell pellet was resuspended in a 50% methanol solution. Total expressed product was determined using HPLC analysis. The results are provided in Table V.

Table III

Exemplary Plasmids Containing Heterologous Promoters

| pHKY(HEPS) | pHKY(PUC) | pHKY(TN5) | pHJL(HEP/TN/302) | pHJL(HEP/TN/401) | pHKY(HEP/hGH) |
|---|---|---|---|---|---|
| pHKY173 | pHKY192 | pHKY195 | pHJL435 | pHJL389 | pHKY210 |
| pHKY177 | pHKY193 | pHKY196 | pHJL436 | pHJL390 | pHKY214 |
| pHKY179 | pHKY203 | pHKY198 | pHJL437 | pHJL392 | pHKY216 |
| pHKY180* | pHKY208 | pHKY199 | pHJL438 | pHJL396 | pHKY213 |
| pHKY183* | | pHKY205 | | | |
| pHKY183b | pHKY190 | pHKY206 | | pHJL397 | pHKY209 |
| pHKY184 | pHKY191 | pHKY200 | pHJL439 | pHJL393 | pHKY215 |
| pHKY185 | pHKY194 | pHKY201 | pHJL440 | pHJL394 | pHKY211 |

*Sequence analysis revealed that plasmid pHKY180 is equivalent to pHKY183 (see text).

EP 0 456 507 A2

Table IV

Levels of Met-Asp-HGH Expressed Using $\lambda_{pL}$ and Heterologous Promoters

| pHKY(HEP/hGH) | Dry Cell Weight(g/L) | HPLC Potency(μg/ml) | Specific Activity(%)‡ |
|---|---|---|---|
| pCZR340 | 1.51 | 124.2 | 8.2 |
| pHKY209 | 2.5 | 152.5 | 6.1 |
| pHKY210 | 2.1 | 64.5 | 3.1 |
| pHKY214 | 1.6 | 138.0 | 8.6 |
| pHKY215 | 2.2 | 50.5 | 2.4 |
| pHKY216 | 2.3 | 94.5 | 4.1 |

*Average of 6 replications

‡Percentage is Product per dry weight

Table V

Levels of Met-Asp-HGH Expressed Using $\lambda_{pL}$ and
Heterologous Promoters in Shake Flask Fermentations

| pHKY(HEP/hGH) | Dry Weight(mg/ml) | HPLC Potency(mg/ml) | Specific Activity(%)‡ |
|---|---|---|---|
| pHJL52* | 0.67 | .00025 | 0.04 |
| pCZR340 (control) | 0.84 | .01744 | 2.10 |
| pHKY146 | 0.81 | 0.03136 | 3.90 |
| pHKY209 | 0.88 | 0.02844 | 3.20 |
| pHKY210 | 0.85 | 0.01911 | 2.25 |
| pHKY211 | 0.73 | 0.00131 | 0.18 |
| pHKY212 | 0.72 | 0.00194 | 0.27 |
| pHKY213 | 0.83 | 0.00425 | 0.51 |
| pHKY214 | 0.54 | 0.03385 | 6.27 |
| pHKY215 | 0.76 | 0.01081 | 1.41 |
| pHKY216 | 0.47 | 0.1712 | 3.60 |

*Promoterless Construct
‡Percentage in Product Per Dry Weight

These data show that the heterologous promoters of the invention are capable of expressing a heterologous protein in E. coli. Further, these data show that the promoters of the invention are able to promote transcription and expression at levels at or exceeding that of the strongest known naturally occurring E. coli promoter, $\lambda_{pL}$.

27

## Claims

1. A DNA sequence which is functional as a promoter in a heterologous host cell, said sequence derived from an organism selected from the group consisting of <u>Streptomyces griseofuscus</u>, an actinophage, and mutants, derivatives, or variants thereof.

2. A DNA promoter sequence as claimed in Claim 1 in which the actinophage is selected from the group consisting of FP43, FP22r4, and mutants, derivatives, or variants thereof.

3. A promoter sequence which is functional in a heterologous host cell and which is contained within a DNA sequence selected from the group consisting of

i)

```
  1   TCTAGAGTCG   AACAATTCTA   TACATTTTTC   CACCACGCTG   TCTAATGCCA
 51   TTAGCCTCGT   TAACGAAGCG   AACATCCGTC   ACTACATAAT   TCCTATTCAT
101   ATGCATGGAG   TTGAATGTGG   CATCTGCCCA   AATTTCATTT   CCGAGAATAC
151   CGCGAACACA   GTTAGTACCA   ACTACTTGCA   TCCATCTTCT   GATATCCTGA
201   GCCCAAATTG   TATCTTTGTA   CCCATTCCAT   CCGTACCTAT   CGAGACGCGG
251   AGCCAAACCT   ACGTCGACCT   GCAGCCCAAG   CTT;
```

ii)

```
  1   TCTAGAGTCG   AACAATTCTA   TACATTTTTC   CACCACGCTG   TCTAATGCCA
 51   TTAGCCTCGT   TAACGAAGCG   AACATCCGTC   ACTACATAAT   TCCTATTCAT
101   ATGCATGGAG   TTGAATGTGG   CATCTGCCCA   AATTTCATTT   CCGAGAATAC
151   CGCGAACACA   GTTAGTACCA   ACTACTTGCA   TCCATCTTCT   GATATCCTGA
```

```
201  GCCCAAATTG  TATCTTTGTA  CCCATTCCAT  CCGTACCTAT  CGATTACATC
251  CCGCAAATTT  GCAATCATTC  CATTACAGTT  TAGTGGGGGA  TTCATCTCAT
301  ACATGAATTC  TCTCAATTTG  TCAGCGAAGG  CAATACGGCG  AAAGCCGATG
351  CTCTCCAGAG  CATCGGCTGC  CGAATCCTTA  CCTGACCTAG  CGTATCCAGC
401  CAAGCCGATA  ATCATTTTAC  ATATACCTCA  AAATCTTGTA  TGCCTCTCGA
451  CCTGCAGCCC  AAGCTT;
```

iii)

```
  1  TCTAGAGTCG  ACCTTGGCTA  CGTCCTCCCA  ATTAACGCCA  TCCTCAGCGA
 51  AAGCAGCAAT  AATTACAGCC  TTCGTGTCAC  TTGGCTTTAC  ATCTACGCCG
101  TAATCGTCGG  CAATCTTAAG  AAGGTCTTCC  TTCTTTAGTG  TATCAAAACT
151  CATGGTATTT  GGTATTCCTC  CTATTGTGTT  TTCATTATAG  CAGACTGTTC
201  GTTCAACGAC  AAAACCCCCG  CT;
```

iv)

```
  1  TCTAGAGTCG  AGTCCTGTCG  GGCGTACTTT  ATCGTTAATT  ACAAAGGGGA
 51  ATGTAATGGG  TTACACGCGA  GTCCGCAAGG  GTGACAATGT  TTTTGTCCAG
101  AAGGGTCCGG  ACAA;
```

v)

```
  1  TCTAGAGTCG  AGTCCTGTCG  GGCGTACTTT  ATCGTTAATT  ACAAAGGGGA
 51  ATGTAATGGG  TTACACGCGA  GTCCGCAAGG  GTGACAATGT  TTTTGTCCAG
101  AAGGGTCCGG  ACAAGGGCAA  GAAGTTGAAG  GTCACGGAAG  AGGGCAAGGG
151  TATGAACCGG  GATTACATCA  AGTTGTCGAC  CTGCAGCCCA  AGCTT;
```

vi)

```
  1   TCTAGAGTCG  ACAAACAATC  TCAACAAGGT  TTGACTCTTC  CTGGCGATGA
 51   ACCGCAAAGG  CCATGTCAGC  ATCGTATTCA  ATTGCCTTGG  ACCATGCAAC
101   CTGACTCATC  ATCGGAGGAG  AATCACGGTC  GGAAAGGTCT  GACTGAGTTG
151   CAGCCGTAAT  ATCAATCAGC  GGGATATTGT  TTGTAACCGC  AAG;
```

vii)

```
  1   TCTAGAGTCG  ACACCAAGAC  GCTCAGCGTT  CTTGTTCACT  GCATCAGGTG
 51   CCAACCATAC  CTTGTCGTTG  ACCATCATGT  CAAACGCCCG  GAATTCCAGA
101   CGCTCA;
```

viii)

```
  1   TCTAGAGTCG  AACGCAGGAA  TGCTCAGATA  CTCGTAGAAG  TAATGCCTCT
 51   CATCTCCTCG  CTTAGGATTG  TCCACAAAAG  TGGCCGCTCC  AATTGCATTC
101   AGAGACTTGA  GAACCCTGAT  ATTTAGCCCA  TTACCCTTCT  CCATAACCTT
151   TGCCTCTAGG· GCAGCGTAAT  TCTCGTAAGG  TCGTGAGTCA  ATCAACTTCT
201   TTCCGAGATT  ATCCGAAATG  AACTTGATGT  TTGCCAGTCC  GAATCGA;
```

4. A DNA promoter sequence as claimed in any one of Claims 1 to 3 wherein said sequence is functional in <u>Escherichia coli</u>.

5. A recombinant DNA vector comprising a DNA promoter sequence as claimed in any one of Claims 1 to 4.

6. A recombinant DNA vector selected from the group consisting of pHKY173, pHKY177, pHKY179, pHKY180, pHKY183, pHKY183b, pHKY184, pHKY185, pHKY192, pHKY193, pHKY203, pHKY208, pHKY190, pHKY191, pHKY194, pHKY195, pHKY196, pHKY198, pHKY199, pHKY205, pHKY206, pHKY200, pHKY201, pHJL435, pHJL436, pHJL437, pHJL438, pHJL439, pHJL440, pHJL389, pHJL390, pHJL392, pHJL396, pHJL397, pHJL393, pHJL394, pHKY210, pHKY214, pHKY216, pHKY213, pHKY209, pHKY215, and pHKY211.

7. A host cell transformed with a recombinant DNA vector comprising a DNA promoter sequence as claimed in any one of Claims 1 to 4.

8. A host cell as claimed in Claim 7 which is <u>Escherichia coli</u>.

9. A method for expressing in <u>Escherichia coli</u> a functional polypeptide which comprises
   (a) transforming an <u>E. coli</u> host cell with a recombinant DNA expression vector, said vector comprising:
       (1) a promoter as claimed in any one of Claims 1 to 4;
       (2) a translational activating sequence; and
       (3) a gene encoding a functional polypeptide; and,
   (b) culturing the transformed <u>E. coli</u> cell under conditions suitable for expression of said polypeptide.

10. A method as claimed in Claim 9 in which the gene encoding a functional polypeptide is selected from the group consisting of genes encoding human preproinsulin, human proinsulin, a human insulin, human insulin A-chain, human insulin B-chain, a non-human insulin, a non-human preproinsulin, a non-human proinsulin, a human growth hormone, a bovine growth hormone, a porcine growth hormone, a tissue plasminogen activator, a Protein C, a Protein S, a human interferon, a non-human interferon, an immunoglobulin, an immunoglobulin fragment, a polypeptide hormone, a polypeptide enzyme, and a polypeptide antigen.

**11.** A method as claimed in Claim 9 or 10 in which the gene encoding a functional polypetide is a gene encoding Met-Asp-human growth hormone.

**Claims for the following Contracting States : ES and GR**

**1.** A method, for expressing in <u>Escherichia coli</u> a functional polypeptide, which comprises
    a) transforming an <u>E. coli</u> host cell with a recombinant DNA expression vector, said vector comprising;
        1) a promoter sequence derived from an organism selected from the group consisting of <u>Strep-tomyces griseofuscus</u>, an actinophage, and mutants, derivatives, or variants thereof;
        2) a translational activating sequence; and
        3) a gene encoding a functional polypeptide; and,
    b) culturing the transformed <u>E. coli</u> cell under conditions suitable for expression of said polypeptide.

**2.** A process according to Claim 1 where the promoter sequence is contained within a DNA sequence selected from the group consisting of

  i)

| | | | | | |
|---|---|---|---|---|---|
| 1 | TCTAGAGTCG | AACAATTCTA | TACATTTTTC | CACCACGCTG | TCTAATGCCA |
| 51 | TTAGCCTCGT | TAACGAAGCG | AACATCCGTC | ACTACATAAT | TCCTATTCAT |
| 101 | ATGCATGGAG | TTGAATGTGG | CATCTGCCCA | AATTTCATTT | CCGAGAATAC |
| 151 | CGCGAACACA | GTTAGTACCA | ACTACTTGCA | TCCATCTTCT | GATATCCTGA |
| 201 | GCCCAAATTG | TATCTTTGTA | CCCATTCCAT | CCGTACCTAT | CGAGACGCGG |
| 251 | AGCCAAACCT | ACGTCGACCT | GCAGCCCAAG | CTT; | |

ii)

```
  1   TCTAGAGTCG   AACAATTCTA   TACATTTTTC   CACCACGCTG   TCTAATGCCA
 51   TTAGCCTCGT   TAACGAAGCG   AACATCCGTC   ACTACATAAT   TCCTATTCAT
101   ATGCATGGAG   TTGAATGTGG   CATCTGCCCA   AATTTCATTT   CCGAGAATAC
151   CGCGAACACA   GTTAGTACCA   ACTACTTGCA   TCCATCTTCT   GATATCCTGA
201   GCCCAAATTG   TATCTTTGTA   CCCATTCCAT   CCGTACCTAT   CGATTACATC
251   CCGCAAATTT   GCAATCATTC   CATTACAGTT   TAGTGGGGGA   TTCATCTCAT
301   ACATGAATTC   TCTCAATTTG   TCAGCGAAGG   CAATACGGCG   AAAGCCGATG
351   CTCTCCAGAG   CATCGGCTGC   CGAATCCTTA   CCTGACCTAG   CGTATCCAGC
401   CAAGCCGATA   ATCATTTTAC   ATATACCTCA   AAATCTTGTA   TGCCTCTCGA
451   CCTGCAGCCC   AAGCTT;
```

iii)

```
  1   TCTAGAGTCG   ACCTTGGCTA   CGTCCTCCCA   ATTAACGCCA   TCCTCAGCGA
 51   AAGCAGCAAT   AATTACAGCC   TTCGTGTCAC   TTGGCTTTAC   ATCTACGCCG
101   TAATCGTCGG   CAATCTTAAG   AAGGTCTTCC   TTCTTTAGTG   TATCAAAACT
151   CATGGTATTT   GGTATTCCTC   CTATTGTGTT   TTCATTATAG   CAGACTGTTC
201   GTTCAACGAC   AAAACCCCCG   CT;
```

iv)

```
  1   TCTAGAGTCG   AGTCCTGTCG   GGCGTACTTT   ATCGTTAATT   ACAAAGGGGA
 51   ATGTAATGGG   TTACACGCGA   GTCCGCAAGG   GTGACAATGT   TTTTGTCCAG
101   AAGGGTCCGG   ACAA;
```

v)

```
  1   TCTAGAGTCG  AGTCCTGTCG  GGCGTACTTT  ATCGTTAATT  ACAAAGGGGA
 51   ATGTAATGGG  TTACACGCGA  GTCCGCAAGG  GTGACAATGT  TTTTGTCCAG
101   AAGGGTCCGG  ACAAGGGCAA  GAAGTTGAAG  GTCACGGAAG  AGGGCAAGGG
151   TATGAACCGG  GATTACATCA  AGTTGTCGAC  CTGCAGCCCA  AGCTT;
```

vi)

```
  1   TCTAGAGTCG  ACAAACAATC  TCAACAAGGT  TTGACTCTTC  CTGGCGATGA
 51   ACCGCAAAGG  CCATGTCAGC  ATCGTATTCA  ATTGCCTTGG  ACCATGCAAC
101   CTGACTCATC  ATCGGAGGAG  AATCACGGTC  GGAAAGGTCT. GACTGAGTTG
151   CAGCCGTAAT  ATCAATCAGC  GGGATATTGT  TTGTAACCGC  AAG;
```

vii)

```
  1   TCTAGAGTCG  ACACCAAGAC  GCTCAGCGTT  CTTGTTCACT  GCATCAGGTG
 51   CCAACCATAC  CTTGTCGTTG  ACCATCATGT  CAAACGCCCG  GAATTCCAGA
101   CGCTCA;
```

viii)

```
  1   TCTAGAGTCG  AACGCAGGAA  TGCTCAGATA  CTCGTAGAAG  TAATGCCTCT
 51   CATCTCCTCG  CTTAGGATTG  TCCACAAAAG  TGGCCGCTCC  AATTGCATTC
101   AGAGACTTGA  GAACCCTGAT  ATTTAGCCCA  TTACCCTTCT  CCATAACCTT
151   TGCCTCTAGG  GCAGCGTAAT  TCTCGTAAGG  TCGTGAGTCA  ATCAACTTCT
201   TTCCGAGATT  ATCCGAAATG  AACTTGATGT  TTGCCAGTCC  GAATCGA;
```

3. A process as claimed in Claim 1 or Claim 2 where E. coli is transformed with a recombinant DNA vector selected from the group consisting of pHKY173, pHKY177, pHKY179, pHKY180, pHKY183, pHKY183b, pHKY184, pHKY185, pHKY192, pHKY193, pHKY203, pHKY208, pHKY190, pHKY191, pHKY194, pHKY195, pHKY196, pHKY198, pHKY199, pHKY205, pHKY206, pHKY200, pHKY201, pHJL435, pHJL436, pHJL437, pHJL438, pHJL439, pHJL440, pHJL389, pHJL390, pHJL392, pHJL396, pHJL397, pHJL393, pHJL394, pHKY210, pHKY214, pHKY216, pHKY213, pHKY209, pHKY215, and pHKY211.

4. A process as claimed in Claim 1 where the gene encoding a functional polypeptide is a gene encoding Met-Asp-human growth hormone.

# FIG.I

RESTRICTION SITE AND FUNCTION MAP
OF PLASMID pHJL35
(~6.0 kb)

# FIG.2

RESTRICTION SITE AND FUNCTION MAP
OF GENERALIZED PLASMID
pHKY(HEPS)

# FIG.3

## RESTRICTION SITE AND FUNCTION MAP OF
## PLASMID pHJL45
## (~3.9 kb)

# FIG.4

## RESTRICTION SITE AND FUNCTION MAP OF GENERALIZED PLASMID pHKY(TN5)

# FIG.5

RESTRICTION SITE AND FUNCTION MAP
OF PLASMID pHJL48
(~6.3 kb)

# FIG.6

RESTRICTION SITE AND FUNCTION MAP
OF PLASMID pHJL302
(~5.1 kb)

# FIG.7

## RESTRICTION SITE AND FUNCTION MAP OF PLASMID pHJL400 (~5.9 kb)

# FIG.8

RESTRICTION SITE AND FUNCTION MAP
OF PLASMID pHJL401
(~5.9 kb)

# FIG.9

RESTRICTION SITE AND FUNCTION MAP OF
PLASMID pHJL433
(~8.4 kb)

# FIG.IO

## RESTRICTION SITE AND FUNCTION MAP OF
## PLASMID pHJL379
## (~9.1 kb)

# FIG.11

RESTRICTION SITE AND FUNCTION MAP OF
PLASMID pHJL388
(~9.1 kb)

# FIG.12

RESTRICTION SITE AND FUNCTION MAP OF
GENERALIZED PLASMID
pHJL(HEP/TN/302)

# FIG.13

## RESTRICTION SITE AND FUNCTION MAP OF GENERALIZED PLASMID pHJL(HEP/TN/400)

# FIG.14

RESTRICTION SITE AND FUNCTION MAP OF
GENERALIZED PLASMID pHJL(HEP/TN/401)

# FIG.15

RESTRICTION SITE AND FUNCTION MAP OF
GENERALIZED PLASMID pHKY(PUC)

# FIG.I6

NdeI
XbaI
HindIII
BamHI
NcoI
Met-Asp-hGH
NdeI
omega
Ori
HindIII
BamHI
SstI
EcoRI
Tc-r
cI857
BamHI
EcoRV

RESTRICTION SITE AND FUNCTION MAP OF
PLASMID pHJL52
(~7.8 kb)

# FIG.17

## RESTRICTION SITE AND FUNCTION MAP OF GENERALIZED PLASMID pHKY(HEP/hGH)

SEQUENCE LISTING

(1) GENERAL INFORMATION:

    (i) APPLICANT:Eli Lilly and Company

    (ii) TITLE OF INVENTION:  Heterologous Promoter Systems

    (iii) NUMBER OF SEQUENCES: 8

    (iv) CORRESPONDENCE ADDRESS:

        (A) ADDRESSEE:Mr. C. Mark Hudson

        (B) STREET:Erl Wood Manor

        (C) CITY:Windlesham

        (D) STATE:Surrey GU20 6PH

        (E) COUNTRY:Great Britain

        (F) ZIP:

    (v) COMPUTER READABLE FORM:

        (A) MEDIUM TYPE:Diskette, 3.50 inch, 1.0 Mb storage

        (B) COMPUTER:Macintosh

        (C) OPERATING SYSTEM:Macintosh

        (D) SOFTWARE:Microsoft Word

    (vi) CURRENT APPLICATION DATA:

        (A) APPLICATION NUMBER:

        (B) FILING DATE:

        (C) CLASSIFICATION:

    (vii) PRIOR APPLICATION DATA:

        (A) APPLICATION NUMBER:

        (B) FILING DATE:

(viii) ATTORNEY/AGENT INFORMATION:

   (A) NAME:Mr. C. Mark Hudson

   (B) REGISTRATION NUMBER:307

   (C) REFERENCE/DOCKET NUMBER:X-7520

 (ix) TELECOMMUNICATION INFORMATION:

   (A) TELEPHONE:0276 78441

   (B) TELEFAX:0276 78306

   (C) TELEX:858177


(2) INFORMATION FOR SEQ ID NO: 1:

   (i) SEQUENCE CHARACTERISTICS:

   (A) LENGTH:283 base pairs

   (B) TYPE:nucleotides

      (C) STRANDEDNESS: double

      (D) TOPOLOGY:linear

 (ii) MOLECULE TYPE: DNA

 (iii) SEQUENCE DESCRIPTION: SEQ ID NO:1

```
TCTAGAGTCG  AACAATTCTA  TACATTTTTC  CACCACGCTG  TCTAATGCCA 50
TTAGCCTCGT  TAACGAAGCG  AACATCCGTC  ACTACATAAT  TCCTATTCAT 100
ATGCATGGAG  TTGAATGTGG  CATCTGCCCA  AATTTCATTT  CCGAGAATAC 150
CGCGAACACA  GTTAGTACCA  ACTACTTGCA  TCCATCTTCT  GATATCCTGA 200
GCCCAAATTG  TATCTTTGTA  CCCATTCCAT  CCGTACCTAT  CGAGACGCGG 250
AGCCAAACCT  ACGTCGACCT  GCAGCCCAAG  CTT 283
```

(3) INFORMATION FOR SEQ ID NO:  2:

   (i) SEQUENCE CHARACTERISTICS:

   (A) LENGTH:466 base pairs

   (B) TYPE:nucleotides

      (C) STRANDEDNESSS:double

(D)  TOPOLOGY:linear

(ii)  MOLECULE TYPE:DNA

(iii)  SEQUENCE DESCRIPTION: SEQ ID NO:  2:

```
TCTAGAGTCG  AACAATTCTA  TACATTTTTC  CACCACGCTG  TCTAATGCCA 50
TTAGCCTCGT  TAACGAAGCG  AACATCCGTC  ACTACATAAT  TCCTATTCAT 100
ATGCATGGAG  TTGAATGTGG  CATCTGCCCA  AATTTCATTT  CCGAGAATAC 150
CGCGAACACA  GTTAGTACCA  ACTACTTGCA  TCCATCTTCT  GATATCCTGA 200
GCCCAAATTG  TATCTTTGTA  CCCATTCCAT  CCGTACCTAT  CGATTACATC 250
CCGCAAATTT  GCAATCATTC  CATTACAGTT  TAGTGGGGGA  TTCATCTCAT 300
ACATGAATTC  TCTCAATTTG  TCAGCGAAGG  CAATACGGCG  AAAGCCGATG 350
CTCTCCAGAG  CATCGGCTGC  CGAATCCTTA  CCTGACCTAG  CGTATCCAGC 400
CAAGCCGATA  ATCATTTTAC  ATATACCTCA  AAATCTTGTA  TGCCTCTCGA 450
CCTGCAGCCC  AAGCTT 466
```

(4)  INFORMATION FOR SEQ ID NO:  3:

   (i)  SEQUENCE CHARACTERISTICS:

   (A)  LENGTH:222 base pairs

   (B)  TYPE:nucleotides

      (C)  STRANDEDNESSS:double

      (D)  TOPOLOGY:linear

(ii)  MOLECULE TYPE:DNA

(iii)  SEQUENCE DESCRIPTION: SEQ ID NO:  3:

```
TCTAGAGTCG  ACCTTGGCTA  CGTCCTCCCA  ATTAACGCCA  TCCTCAGCGA 50
AAGCAGCAAT  AATTACAGCC  TTCGTGTCAC  TTGGCTTTAC  ATCTACGCCG 100
TAATCGTCGG  CAATCTTAAG  AAGGTCTTCC  TTCTTTAGTG  TATCAAAACT 150
CATGGTATTT  GGTATTCCTC  CTATTGTGTT  TTCATTATAG  CAGACTGTTC 200
GTTCAACGAC  AAAACCCCCG  CT 222
```

(5)  INFORMATION FOR SEQ ID NO:  4:

   (i) SEQUENCE CHARACTERISTICS:

    (A)  LENGTH:114 base pairs

    (B)  TYPE:nucleotides

     (C)  STRANDEDNESSS:double

     (D)  TOPOLOGY:linear

  (ii) MOLECULE TYPE:DNA          .

  (iii) SEQUENCE DESCRIPTION: SEQ ID NO:   4

```
TCTAGAGTCG  AGTCCTGTCG  GGCGTACTTT  ATCGTTAATT  ACAAAGGGGA 50
ATGTAATGGG  TTACACGCGA  GTCCGCAAGG  GTGACAATGT  TTTTGTCCAG 100
AAGGGTCCGG  ACAA 114
```

(6)  INFORMATION FOR SEQ ID NO: 5:

   (i) SEQUENCE CHARACTERISTICS:

    (A)  LENGTH:195

    (B)  TYPE:nucleotides

     (C)  STRANDEDNESSS:double

     (D)  TOPOLOGY:linear

  (ii) MOLECULE TYPE:DNA

  (iii) SEQUENCE DESCRIPTION: SEQ ID NO: 5

```
TCTAGAGTCG  AGTCCTGTCG  GGCGTACTTT  ATCGTTAATT  ACAAAGGGGA 50
ATGTAATGGG  TTACACGCGA  GTCCGCAAGG  GTGACAATGT  TTTTGTCCAG 100
AAGGGTCCGG  ACAAGGGCAA  GAAGTTGAAG  GTCACGGAAG  AGGGCAAGGG 150
TATGAACCGG  GATTACATCA  AGTTGTCGAC  CTGCAGCCCA  AGCTT 195
```

(7)  INFORMATION FOR SEQ ID NO: 6:

   (i) SEQUENCE CHARACTERISTICS:

    (A)  LENGTH:193

    (B)  TYPE:nucleotides

```
(C) STRANDEDNESSS:double

(D) TOPOLOGY:linear

(ii) MOLECULE TYPE:DNA

(iii) SEQUENCE DESCRIPTION: SEQ ID NO: 6
TCTAGAGTCG  ACAAACAATC  TCAACAAGGT  TTGACTCTTC  CTGGCGATGA 50
ACCGCAAAGG  CCATGTCAGC  ATCGTATTCA  ATTGCCTTGG  ACCATGCAAC 100
CTGACTCATC  ATCGGAGGAG  AATCACGGTC  GGAAAGGTCT  GACTGAGTTG 150
CAGCCGTAAT  ATCAATCAGC  GGGATATTGT  TTGTAACCGC  AAG 193

(8) INFORMATION FOR SEQ ID NO: 7:

    (i) SEQUENCE CHARACTERISTICS:

    (A) LENGTH:106

    (B) TYPE:nucleotides

        (C) STRANDEDNESSS:double

        (D) TOPOLOGY:linear

(ii) MOLECULE TYPE:DNA

(iii) SEQUENCE DESCRIPTION:  SEQ ID NO:7
TCTAGAGTCG  ACACCAAGAC  GCTCAGCGTT  CTTGTTCACT  GCATCAGGTG 50
CCAACCATAC  CTTGTCGTTG  ACCATCATGT  CAAACGCCCG  GAATTCCAGA 100
CGCTCA 106

(9) INFORMATION FOR SEQ ID NO:8:

    (i) SEQUENCE CHARACTERISTICS:

    (A) LENGTH:247 base pairs

    (B) TYPE:nucleotides

        (C) STRANDEDNESSS:double

        (D) TOPOLOGY:linear

(ii) MOLECULE TYPE:DNA
```

(iii) SEQUENCE DESCRIPTION:    SEQ ID NO:8

```
TCTAGAGTCG  AACGCAGGAA  TGCTCAGATA  CTCGTAGAAG  TAATGCCTCT  50
CATCTCCTCG  CTTAGGATTG  TCCACAAAAG  TGGCCGCTCC  AATTGCATTC  100
AGAGACTTGA  GAACCCTGAT  ATTTAGCCCA  TTACCCTTCT  CCATAACCTT  150
TGCCTCTAGG  GCAGCGTAAT  TCTCGTAAGG  TCGTGAGTCA  ATCAACTTCT  200
TTCCGAGATT  ATCCGAAATG  AACTTGATGT  TTGCCAGTCC  GAATCGA  247
```